(19) Europäisches Patentamt
European Patent Office
Office européen des brevets

(11) EP 1 407 757 A1

(12) **EUROPEAN PATENT APPLICATION**

(43) Date of publication:
**14.04.2004 Bulletin 2004/16**

(51) Int Cl.7: **A61K 7/42**, A61K 7/44

(21) Application number: **02405867.9**

(22) Date of filing: **09.10.2002**

(84) Designated Contracting States:
**AT BE BG CH CY CZ DE DK EE ES FI FR GB GR IE IT LI LU MC NL PT SE SK TR**
Designated Extension States:
**AL LT LV MK RO SI**

(71) Applicant: **Ciba Specialty Chemicals Holding Inc.**
**4057 Basel (CH)**

(72) Inventors:
- **Herzog, Bernd**
**79639 Grenzach-Wyhlen (DE)**
- **Stehlin, Albert**
**68128 Rosenau (FR)**

Remarks:
Claims 11 to 23 are deemed to be abandoned due to non-payment of the claims fees (Rule 31 (2) EPC).

(54) **Preparation of micronised UV absorbers**

(57) Disclosed is a method of producing a cosmetic composition comprising a micronised or-, ganic UV absorber (a), which method comprises grinding the organic UV absorber, in coarse particle form, in a grinding apparatus, in the presence of a composition, comprising

(i) 0.1 to 99,9%, preferably 1 to 40 % by weight, based on the micronised insoluble organic UV absorber, of a dispersing agent (b); and
(ii) 0.1 to 99.9 % preferably 1 to 40 % by weight, based on the micronised organic UV absorber, of an anionic, cationic or amphoteric UV absorber (c).

EP 1 407 757 A1

## Description

The present invention relates o cosmetic and dermatological formulations, most preferably light screen formulations.

The harmful effects of the UV radiation for the human skin are well known. Depending on the wavelength the sunbeams have different effects:

- the UV-C radiation (wave length <290 nm) is normally absorbed by the ozone layer in the stratosphere and has no physiological significance.
- radiation from 290 to 320 nm, the so called UV-B-range, is primarily responsible for sunburn, while
- the UV-A radiation in the range from 320 to 400 nm is responsible for skin aging and also contributes to sunburn.

It is well known, that specific organic UV-filters, for example sparingly soluble benzotriazole- and triazine compounds, have distinct UV-filter properties and cover a broad UV-spectrum. It is also known, that these sparingly soluble filters can be dispersed with micronization processes and are therefore accessible for an effective sunscreen formulation.

An effective micronization process of said UV-filters can only be carried out in presence of a dispersing agent. It is desired to keep the concentration of the dispersing agent as low as possible.

Surprisingly it was found that the concentration of the dispersing agent can be diminished using a ionic UV absorber as co-dispersant. Simultaneously the efficiency of the micronization stays constant or can even be improved as well as the sun protection properties of the UV absorber formulation.

Therefore, the present invention relates to a method of producing a cosmetic composition comprising a micronised organic UV absorber (a), which method comprises grinding the organic UV absorber, in coarse particle form, in a grinding apparatus, in the presence of a composition, comprising

(i) 0.1 to 99,9%, preferably 1 to 40 % by weight, based on the micronised insoluble organic UV absorber, of a dispersing agent (b); and

(ii) 0.1 to 99.9 % preferably 1 to 40 % by weight, based on the micronised organic UV absorber, of an anionic, cationic or amphoteric UV absorber (c).

Organic UV filters suitable for use according to the invention (component (a)) are in some cases sparingly soluble, compounds, for example triazine derivatives, especially hydroxy-phenyltriazine compounds or benzotriazole derivatives, amides containing a vinyl group, cinnamic acid derivatives, Fischer base derivatives, diphenyl malonic acid dinitriles, oxalyl amides, camphor derivatives, diphenyl acrylates, para-aminobenzoic acid (PABA) and derivatives thereof, salicylates, benzophenones and further classes of substances known as UV filters.

Preferred triazine derivatives suitable for use according to the invention correspond to formula

(1) 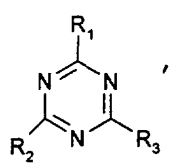 ,

| | |
|---|---|
| $R_1$, $R_2$ and $R_3$ | are each independently of the others hydrogen; OH; $C_1$-$C_{18}$alkoxy; $-NH_2$; $-NH-R_4$; $-N(R_4)_2$; $-OR_4$, |
| $R_4$ | is $C_1$-$C_5$alkyl; phenyl; phenoxy; anilino; pyrrolo, wherein phenyl, phenoxy, anilino and pyrrolo are unsubstituted or may be substituted by one, two or three OH groups, carboxy, $-CO-NH_2$, $C_1$-$C_5$alkyl or $C_1$-$C_5$alkoxy; a methylidene-camphor group; a group of formula $-(CH=CH)_mC(=O)-OR_4$; a group of formula |

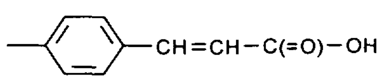

or a corresponding alkali metal, ammonium, mono-, di- or tri-$C_1$-$C_4$alkylammonium, mono-, di- or tri-$C_2$-$C_4$alkanolammonium salt, or a $C_1$-$C_3$alkyl ester thereof; or a radical of formula (1a)

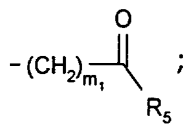

| | |
|---|---|
| $R_5$ | is hydrogen; $C_1$-$C_5$alkyl unsubstituted or substituted by one or more OH groups; $C_1$-$C_5$alkoxy; amino; mono- or di-$C_1$-$C_5$alkylamino; M; a radical of formula |

(1b) 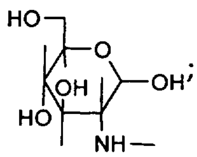 ; (1c) 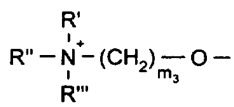 ; (1d) 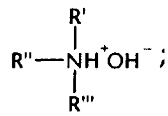 ;

or

(1e) 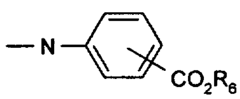 ;

wherein

| | |
|---|---|
| R', R'' and R''' | are each independently of the others $C_1$-$C_{14}$alkyl unsubstituted or substituted by one or more OH groups; |
| $R_6$ | is hydrogen; M; $C_1$-$C_5$alkyl; or a radical of formula $-(CH_2)_{m_2}-O-T_1$; |
| M | is a metal cation; |
| $T_1$ | is hydrogen; or $C_1$-$C_8$alkyl; |
| m | is 0 or 1; |
| $m_2$ | is from 1 to 4; and |
| $m_3$ | is from 2 to 14. |

Further preferred triazine derivatives suitable for use according to the invention correspond to formula

(2) 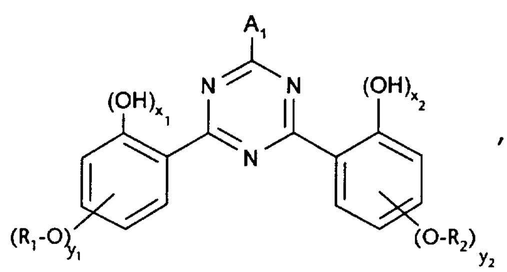 ,

wherein

| | |
|---|---|
| $R_1$ and $R_8$ | are each independently of the other $C_1$-$C_{18}$alkyl; $C_2$-$C_{18}$alkenyl; a radical of formula $-CH_2-CH(-OH)-CH_2-O-T_1$ ; or |
| $R_1$ and $R_2$ | are a radical of formula (2a) |

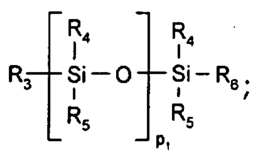

$R_3$ is a direct bond; a straight-chain or branched $C_1$-$C_4$alkylene radical or a radical of formula

$-C_{m_1}H_{2m_1}-O-$ ;

$R_4$, $R_5$ and $R_6$ are each independently of the others $C_1$-$C_{18}$alkyl; $C_1$-$C_{18}$alkoxy or a radical of formula

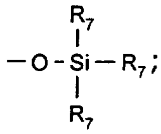

$R_7$ is $C_1$-$C_5$alkyl;
$m_1$ is from 1 to 4;
$p_1$ is from 0 to 5;
$A_1$ is a radical of formula

(2b) 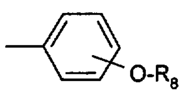 ; (2c) 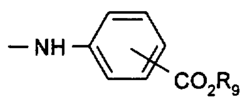 ;

or of formula

(2d) 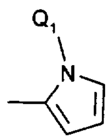 ;

$R_8$ is hydrogen; $C_1$-$C_{10}$alkyl, $-(CH_2CHR_{10}-O)_{n_1}-R_9$; or a radical of formula $-CH_2-CH(-OH)-CH_2-O-T_1$;
$R_9$ is hydrogen; M; $C_1$-$C_5$alkyl; or a radical of formula $-(CH_2)_{m_2}-O-(CH_2)_{m_3}-T_1$;
$R_{10}$ is hydrogen; or methyl;
$T_1$ is hydrogen; or $C_1$-$C_8$alkyl;
$Q_1$ is $C_1$-$C_{18}$alkyl;
M is a metal cation;
$m_2$ and $m_3$ are each independently of the other from 1 to 4;
$n_1$ is from 1 to 16;
$x_1$ and $x_2$, independently from each other are 0 or 1; and
$y_1$ and $y_2$, independently from each other are a number from 1 to 3.

Very especially preferred triazine derivatives of formula (2) correspond to formulae

(2a) 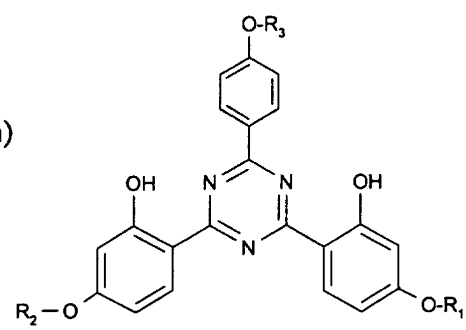 ; (2b) 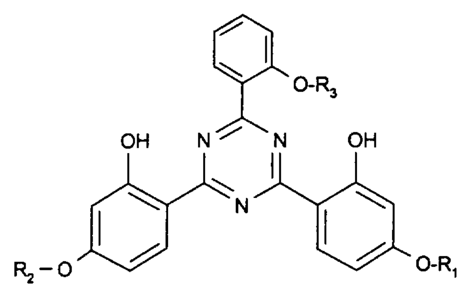 ;

(2c) 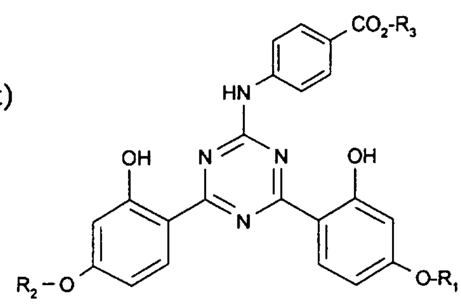 ; and (2d) 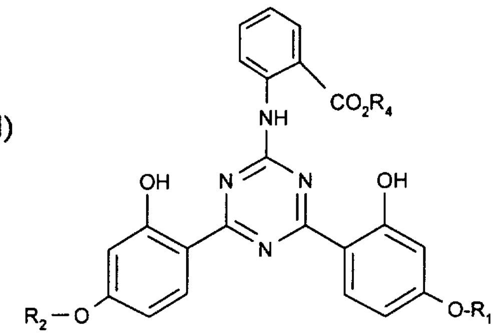 ;

wherein

$R_1$ and $R_2$ are each independently of the other $C_3$-$C_{18}$alkyl; or $-CH_2$-$CH(-OH)$-$CH_2$-O-$T_1$;
$R_3$ is $C_1$-$C_{10}$alkyl or a radical of formula

(2a₁) 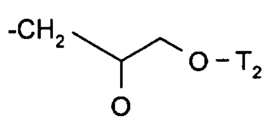 or (2a₂) 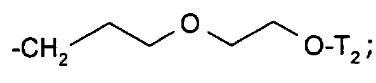

$R_4$ is hydrogen; M; $C_1$-$C_5$alkyl; -NH-$C_1$-$C_5$alkyl; preferably -NH-tert-alkyl; or a radical of formula $-(CH_2)_m$-O-$T_2$;
$T_1$ and $T_2$ are each independently of the other hydrogen; or $C_1$-$C_5$alkyl; and
m is from 1 to 4.

Of very special interests are compounds of formulae (2a) and (2b) wherein

$R_1$ and $R_2$ are each independently of the other $C_1$-$C_{18}$alkyl; or $-CH_2$-$CH(-OH)$-$CH_2$-O-$T_1$;
$R_3$ is $C_1$-$C_{10}$alkyl;

and compounds of formulae (2c) and (2d) wherein

$R_1$ and $R_2$ are each independently of the other $C_1$-$C_{18}$alkyl or $-CH_2$-$CH(-OH)$-$CH_2$-O-$T_1$; and
$T_1$ is hydrogen; or $C_1$-$C_5$alkyl.

Of very great interest are triazine compounds of formulae (2a) - (2d) wherein $R_1$ and $R_{28}$ have the same meaning.

Further interesting triazine compounds suitable for use according to the invention correspond to formula

(3) 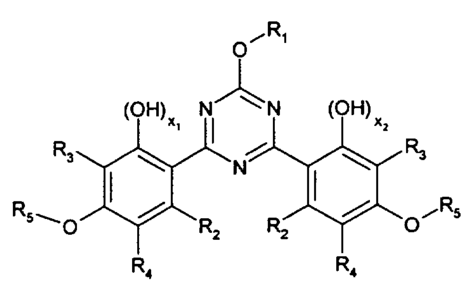

wherein

$R_1$ is $C_1$-$C_{30}$alkyl; $C_2$-$C_{30}$alkenyl; $C_5$-$C_{12}$cycloalkyl unsubstituted or mono- or poly-substituted by $C_1$-$C_5$alkyl; $C_1$-$C_5$alkoxy-$C_1$-$C_{12}$alkyl; amino-$C_1$-$C_{12}$alkyl; $C_1$-$C_5$monoalkylamino-$C_1$-$C_{12}$alkyl; $C_1$-$C_5$dialkylamino-$C_1$-$C_{12}$alkyl; a radical of formula (3a)

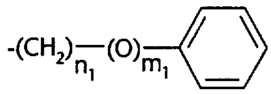

; or (3b)

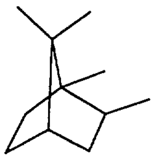 ;

wherein

| | |
|---|---|
| $R_2$, $R_3$ and $R_4$ | are each independently of the others hydrogen, -OH; $C_1$-$C_{30}$alkyl, $C_2$-$C_{30}$alkenyl, |
| $R_5$ | is hydrogen; or $C_1$-$C_5$alkyl; |
| $m_1$ | is 0 or 1; and |
| $n_1$ | is from 1 to 5; and |
| $X_1$ and x2 | are independently from each other 0 or 1. |

Preferred compounds in the present process are compounds of formula

(3a) 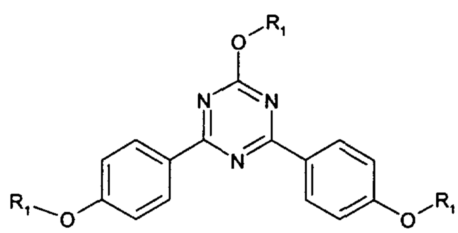 ,

wherein

R is $C_1$-$C_5$alkyl, preferably methyl or ethyl.

Preferred compounds correspond to formula

(4) 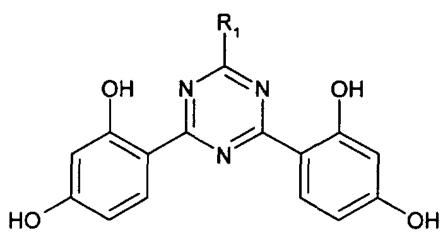 ;

wherein

$R_1$

is 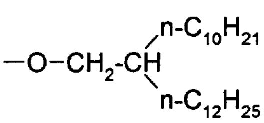 ; $-O-isoC_{18}H_{38}$; 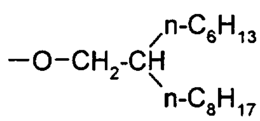 $-O-n-C_{18}H_{37}$;

or

- O-2-ethylhexyl; -O-$(CH_2)_3$-N$(C_2H_5)_2$;

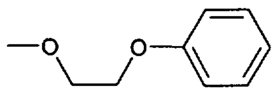 ; 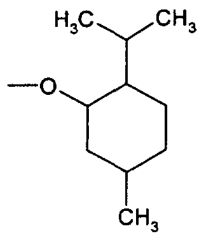 ;

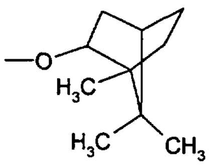 ; 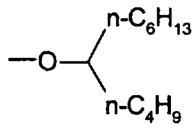 ; -O-n-$C_{18}H_{37}$; 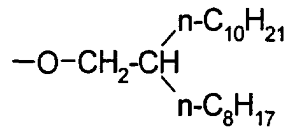 ;

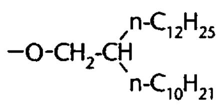 ; 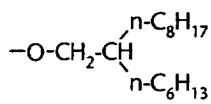 ; 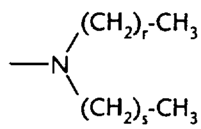

and

r and s are each independently of the other from 0 to 20.

Examples of triazine derivatives suitable for use according to the invention correspond to the formulae

(5) 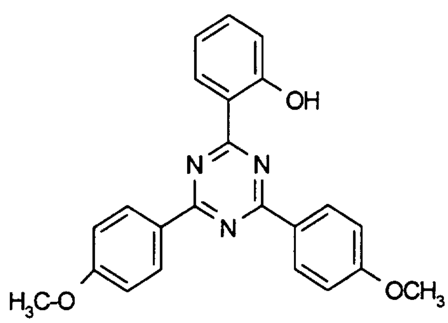 ; (6) 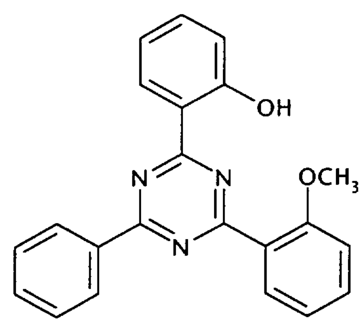 ;

(7) 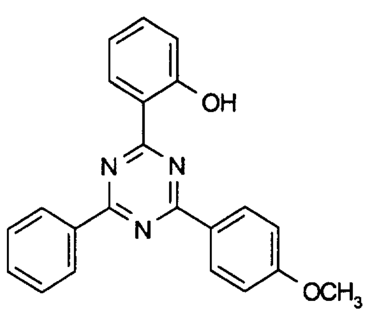 ; (8) 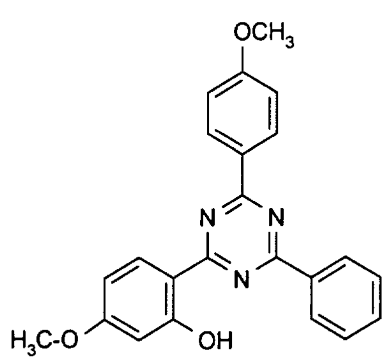 ;

(9) 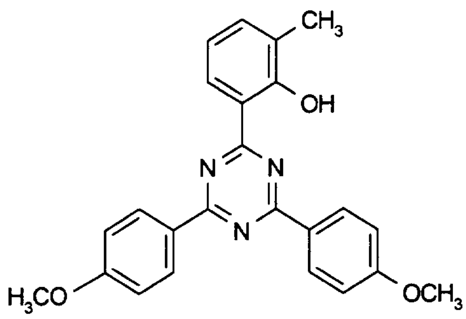 ; (10) 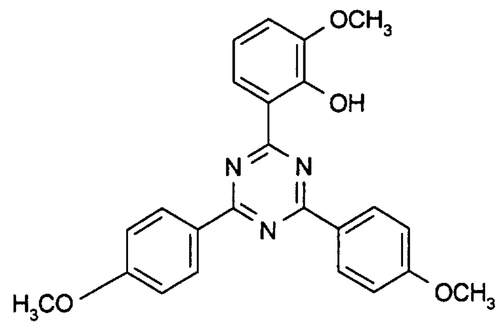 ;

(11) 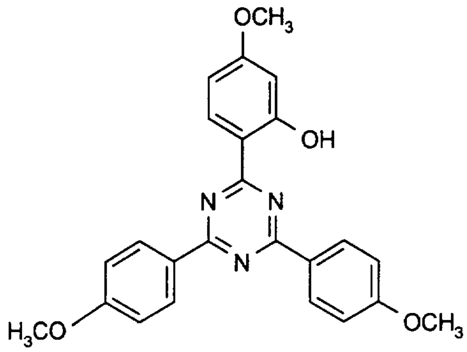 ; (12) 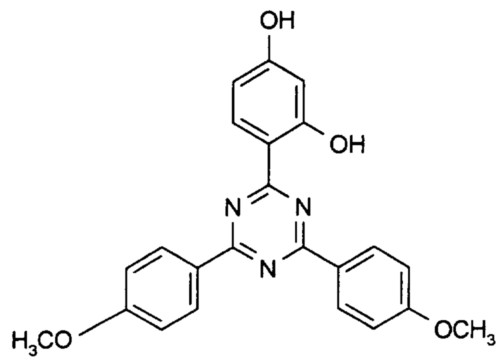 ;

(13) 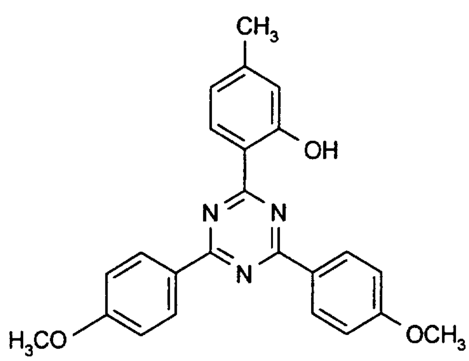 ; (14) 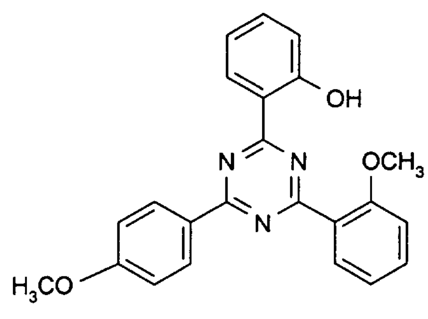 ;

(15) 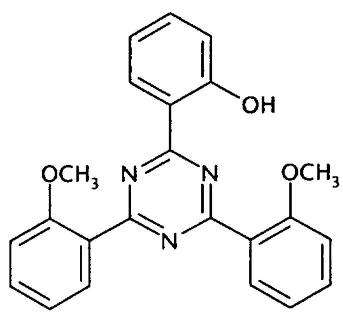 ; (16) 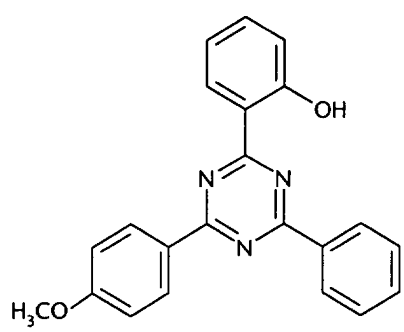 ;

(17) 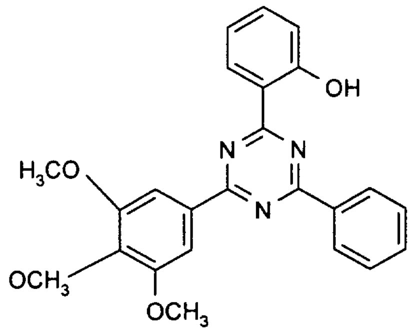 ; (18) 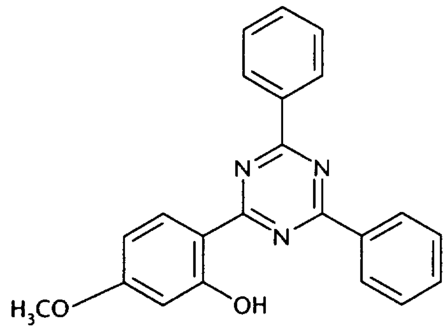 ;

(19) 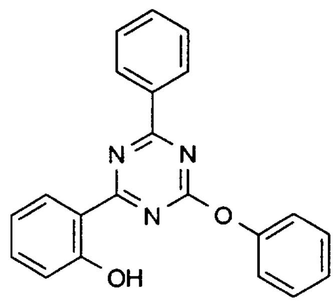 ; (20) 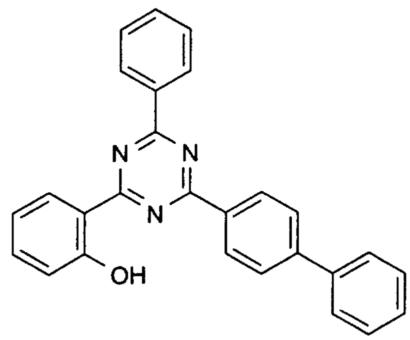 ;

(20a) 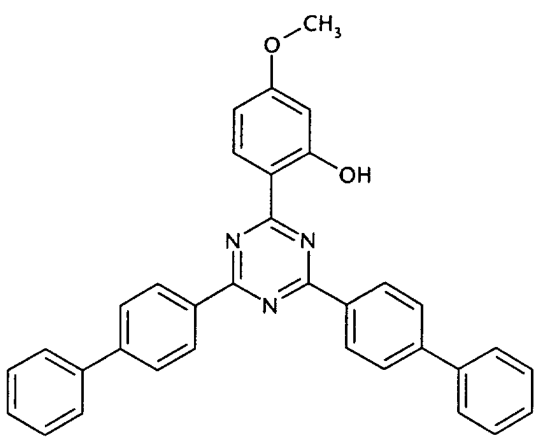 (21) 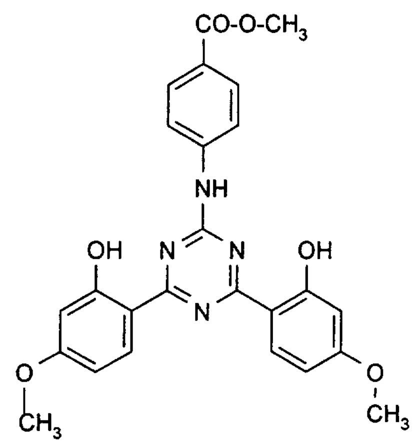 ;

(22) 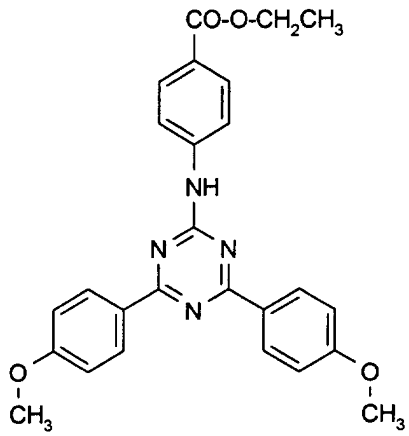 ; (23) 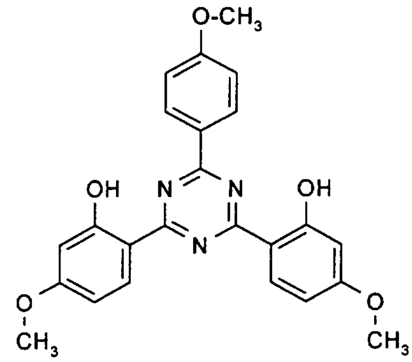 ;

and

(24) 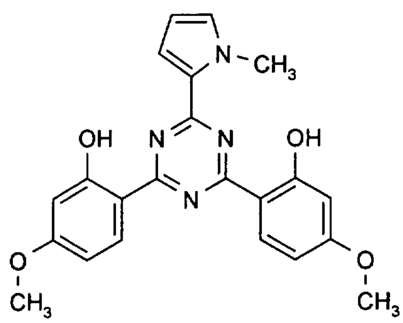 ;

also 2,4,6-tris(diisobutyl-4'-aminobenzalmalonate)-s-triazine and 2,4-bis(diisobutyl-4-aminobenzalmalonate)-6-(4'-aminobenzylidenecamphor)-s-triazine.

Triazine compounds suitable for use according to the invention that are likewise preferred are described in EP-A-654 469, e.g. the compound of formula

(24a) 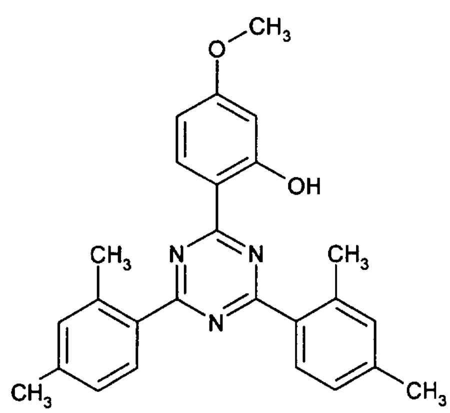 .

Triazine compounds especially suitable for use according to the invention are described, for example, in EP-A-0 818 450, e.g. the compound of formula

(24b) 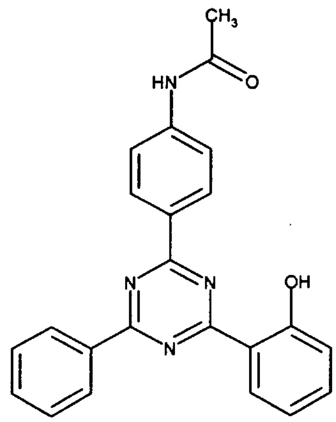 .

Very especially preferred triazine derivatives suitable for use according to the invention correspond to formula

(25) 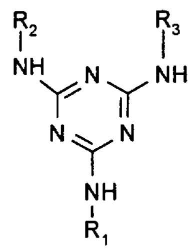 ,

wherein

$R_1$, $R_2$ and $R_3$ are each independently of the others a radical of formula

(25a) 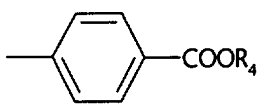 ; (25b) 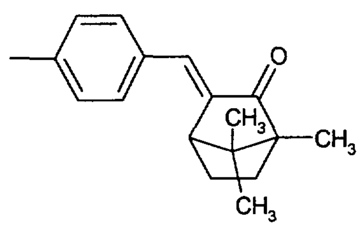 ;

or

(25c) 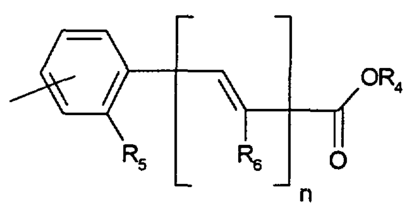 ;

$R_4$ is hydrogen; an alkali metal; an ammonium group $-N^+(R_7)_3$,

$R_7$ is hydrogen, $C_1$-$C_5$alkyl; or a polyoxyethylene radical that has from 1 to 10 ethylene oxide units and the terminal OH group can be etherified with a $C_1$-$C_5$alcohol;

$R_5$ is hydrogen; -OH; or $C_1$-$C_6$alkoxy;

$R_6$ is hydrogen or $-COOR_4$; and

n is 0 or 1.

When $R_4$ is an alkali metal, it is especially potassium or more especially sodium. $(R_7)_3$ is especially a mono-,

di- or tri-$C_1$-$C_4$alkylammonium salt, a mono-, di- or tri-$C_2$-$C_4$alkanolammonium salt or a $C_1$-$C_3$alkyl ester thereof.

When $R_7$ is a $C_1$-$C_3$alkyl group, it is especially a $C_1$-$C_2$alkyl group, more especially a methyl group, and when $R_{33}$ is a polyoxyethylene radical, that radical contains especially from 2 to 6 ethylene oxide units.

Preferred benzotriazole compounds suitable for use according to the invention correspond to formula

(26) 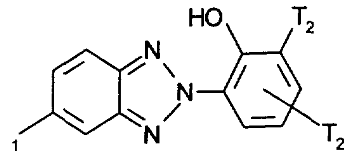 ,

wherein

$T_1$ is $C_1$-$C_{10}$alkyl or preferably hydrogen; and

$T_2$ is $C_1$-$C_{10}$alkyl, preferably tert-butyl, or phenyl-substituted $C_1$-$C_4$alkyl, especially $\alpha,\alpha$-dimethylbenzyl.

A further preferred class of benzotriazole compounds suitable for use according to the invention corresponds to formula

(27) 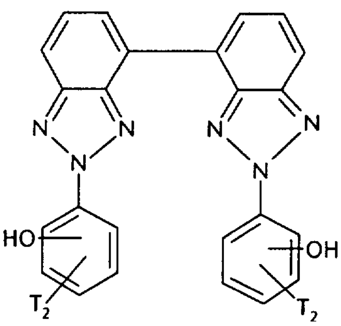 ,

wherein

$T_2$ is $C_1$-$C_{10}$alkyl, preferably iso-octyl, or phenyl-substituted $C_1$-$C_4$alkyl, especially $\alpha,\alpha$-dimethylbenzyl.

Further, especially preferred benzotriazole compounds suitable for use according to the invention correspond to formula

(28) 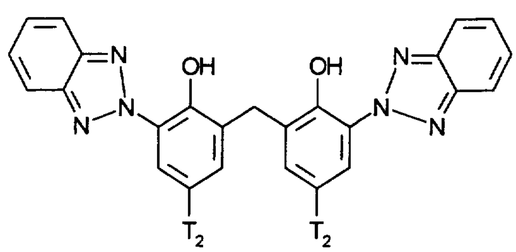 ,

wherein

$T_2$ is as defined for formula (26) and is preferably methyl, tert-butyl or iso-octyl.

Preferred vinyl-group-containing amides suitable for use according to the invention correspond to formula

(1) $$R_1\text{-}(Y)_m\text{-}CO\text{-}C(R_2)=C(R_3)\text{-}N(R_4)(R_5),$$

wherein

| | |
|---|---|
| $R_1$ | is $C_1$-$C_5$alkyl, preferably methyl or ethyl, or phenyl unsubstituted or substituted by one, two or three of the radicals OH, $C_1$-$C_5$alkyl, $C_1$-$C_5$alkoxy and CO-$OR_1$; |
| $R_2$, $R_3$, $R_4$ and $R_5$ | are each independently of the others $C_1$-$C_5$alkyl, preferably methyl or ethyl; or hydrogen; |
| Y | is -NH or -O-; and |
| m | is as defined above. |

Preferred compounds of formula (29) are 4-methyl-3-penten-2-one, ethyl 3-methylamino-2-butenoate, 3-methylamino-1-phenyl-2-buten-1-one and 3-methylamino-1-phenyl-2-buten-1-one.

Preferred cinnamic acid amides suitable for use according to the invention correspond to formula

(30) 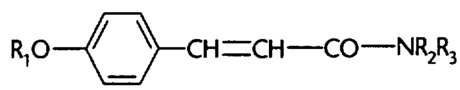 , wherein

$R_1$ is hydrogen or $C_1$-$C_5$alkoxy, preferably methoxy or ethoxy;
$R_2$ is hydrogen or $C_1$-$C_5$alkyl, preferably methyl or ethyl; and
$R_3$ is $-(CONH)_m$-phenyl, wherein m is as defined above and the phenyl group is unsubstituted or substituted by one, two or three of the radicals OH, $C_1$-$C_3$alkyl, $C_1$-$C_3$alkoxy and CO-$OR_5$.
$R_5$ is hydrogen; an alkali metal; an ammonium group $-N^+(R_6)_3$,
$R_6$ is hydrogen, $C_1$-$C_5$alkyl; or a polyoxyethylene radical that has from 1 to 10 ethylene oxide units and the terminal OH group can be etherified with a $C_1$-$C_5$alcohol;
$R_3$ is preferably phenyl, 4-methoxyphenyl or the phenylaminocarbonyl group.

Further preferred cinnamic acid derivatives are 2-ethylhexyl-4-methoxy-cinnamate or - isoamylate or *inter alia* the cinnamic acid derivatives disclosed in US-A-5 601 811 and WO 97/00851.

Preferred Fischer base aldehydes suitable for use according to the invention correspond to formula

(32) 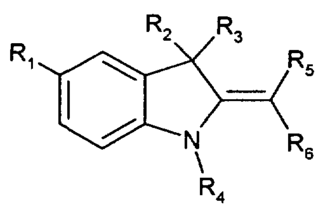 ,

wherein

$R_1$ is hydrogen; $C_1$-$C_5$alkyl; $C_1$-$C_{18}$alkoxy; or halogen;
$R_2$ and $R_3$ independently from each other are $C_1$-$C_8$alkyl; $C_5$-$C_7$cycloalkyl; or $C_6$-$C_{10}$aryl;
$R_4$ is $C_1$-$C_{18}$alkyl or a radical of formula (32a)

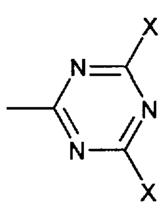 ;

$R_5$ is hydrogen; or a radical of formula

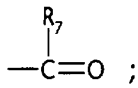

$R_6$ is

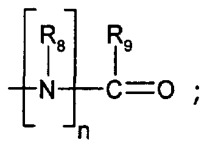

$C_1$-$C_{18}$alkoxy; or a radical of formula (32b)

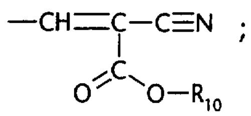

$R_7$ and $R_8$ are each independently of the other hydrogen; or $C_1$-$C_5$alkyl;
$R_9$ is hydrogen; $C_1$-$C_5$alkyl; $C_5$-$C_7$cycloalkyl; phenyl; phenyl-$C_1$-$C_3$alkyl;
$R_{10}$ is $C_1$-$C_{18}$alkyl;
X is Hal; a radical of formula (32c)

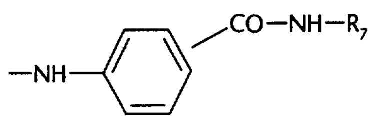

or

(32d) 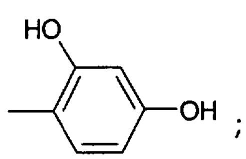

and
n is 0; or 1.

Further compounds that can preferably be used correspond to formula

(33) 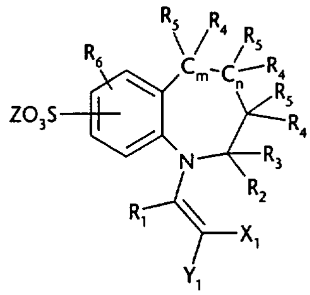 ,

wherein

| | |
|---|---|
| $R_1$, $R_2$, $R_3$, $R_4$, $R_5$ | are each independently of the others hydrogen, $C_1$-$C_8$alkyl or $C_5$-$C_{10}$cycloalkyl; |
| $R_6$ | is hydrogen; $C_1$-$C_8$alkyl; $C_5$-$C_{10}$cycloalkyl; hydroxy; $C_1$-$C_8$alkoxy; $COOR_7$; or $CONR_8R_9$; |
| $R_7$, $R_8$ and $R_9$ | are each independently of the others hydrogen or $C_1$-$C_6$alkyl; |
| X and Y | are each independently of the other hydrogen, -CN; $CO_2R_{10}$; $CONR_{10}R_{11}$; or $COR_{10}$; it being possible for the radicals X and Y additionally to be a $C_1$-$C_8$alkyl radical, a $C_5$-$C_{10}$alkyl radical, especially phenyl, or a heteroaryl radical having 5 or 6 ring atoms, it also being possible for X and Y or |
| $R_{11}$ | together with one of the radicals X and Y to be the radical for completing a 5- to 7-membered ring which may contain up to 3 hetero atoms, especially oxygen and/or nitrogen, it being possible for the ring atoms to be substituted especially by exocyclically double-bonded oxygen (keto oxygen) and/or by $C_1$-$C_8$alkyl and/or by $C_5$-$C_{10}$cycloalkyl radicals and/or to contain C=C double bonds; |
| Z | is hydrogen; ammonium; an alkali metal ion; especially lithium, sodium, potassium, 1/2 equivalent of an alkaline earth metal ion, preferably calcium, magnesium, or the cation of an organic nitrogen base used for neutralisation of the free acid group, |
| $R_{10}$ and $R_{11}$ | are each independently of the other hydrogen, $C_1$-$C_8$alkyl or $C_5$-$C_{10}$cycloalkyl; and |
| n and m | are each independently of the other 0 or 1. |

Preferred diphenylmalonic acid nitriles suitable for use according to the invention correspond to formula

(34) 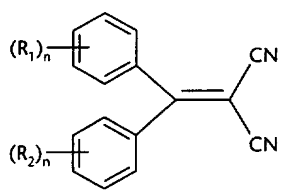 ,

wherein

| | |
|---|---|
| $R_1$ and $R_2$ | are each independently of the other $C_1$-$C_{12}$alkyl; or $C_1$-$C_{12}$alkoxy; and |
| n | is 0-3. |

Further organic UV filters suitable for use according to the invention correspond to formula

(35) 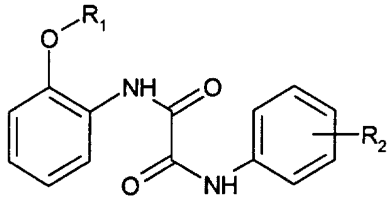 ,

wherein

$R_1$ and $R_2$ are each independently of the other $C_1$-$C_5$alkyl, especially ethyl.

Further preferred chemical compound classes of UV filters suitable for use according to the invention are:

- p-aminobenzoic acid derivatives (PABA), especially 2-ethylhexyl-4-dimethylaminobenzoate;
- salicylic acid derivatives, especially 2-ethylhexyl salicylates; homosalates; and isopropyl salicylates;
- benzophenone derivatives, especially benzophenone-2, -3 and -4;
- dibenzoylmethane derivatives, especially 1-(4-tert-butylphenyl)-3-(4-methoxyphenyl)-propane-1,3-dione or butylmethoxydibenzoylmethane;
- diphenyl acrylates, especially 2-ethylhexyl-2-cyano-3,3-diphenyl acrylate, ethyl 2-cyano-3,3'-diphenyl acrylate and 3-(benzofuranyl)-2-cyanoacrylate;
- 3-imidazol-4-yl acrylate;
- benzofuran derivatives, especially the p-aminophenylbenzofuran derivatives disclosed in EP-A-582 189, US-A-5 338 539 and US-A-5 518 713;
- menthyl o-aminobenzoate.

Anionic UV absorbers (component (c)

anionic absorbers which are based on benzotriazole, benzimidazole, triazine, or benzophenone systems, such as, as merely preferred examples, sulfonated hydroxybenzotriazole (trade name Cibafast®W), sodium 2-hydroxy-4-methoxy-5-sulfo-benzophenone, 2-phenylbenzimidazole-5-sulfonic acid, and sodium 2,2'-dihydroxy-4,4'-dimethoxy-5,5'-disulfobenzophenone;. Hier könnten auch noch weitere Klassen erwähnt werden (siehe unten)

The anionic UV absorbers(component (c)) which are useful for the present process, are selected from the group consisting essentially of sulfonated hydroxybenzotriazole, sodium 2-hydroxy-4-methoxy-5-sulfo-benzophenone, sodium 2,2'-dihydroxy-4,4'-dimethoxy-5,5'-disulfobenzophenone, 2-phenylbenzimidazole-5-sulfonic acid, and mixtures thereof.

Examples of anionic UV absorbers are:

2-Hydroxy-4-methoxy benzophenone-5-sulfonic acid; α-(2-oxoborn-3-ylidene)toluene-4-sulphonic acid and its salts; methyl N,N,N-trimethyl-4-[(4,7,7-trimethyl-3-oxobicyclo[2,2,1]-hept-2-ylidene)methyl]anilinium sulphate; 2-phenyl-1H-benzimidazole-5-sulphonic acid; 3,3'-(1, 4-phenylenedimethylene)bis[7,7-dimethyl-2-oxo-bicyclo[2.2.1]heptane-1-methane-sulfonic acid]; disodium phenyl benzimidazole tetrasulfonate,; 1H-Benzimidazole-4,6-disulfonic acid, 2,2'-(1,4-phenylene)bis-, disodium salt.

Preferred cationic UV absorbers (component (c)) which are useful for the present invention are benzotriazoles of the formula

(1) 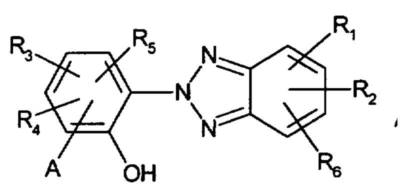

wherein

A is a radical of formula (1a)

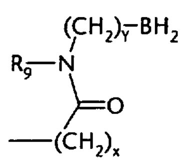 ; (1b) 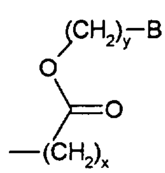 ;

or

(1c) 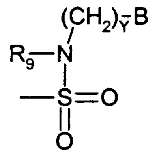 ;

B is a radical of formula (1d)

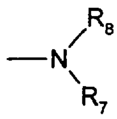 ; (1e) 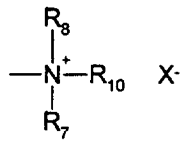 ; (1f) 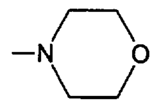 ;

(1g) 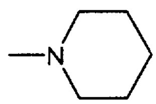 ; (1h) 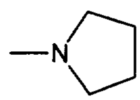 ; (1i) 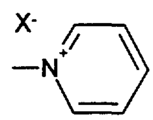 ; (1k) 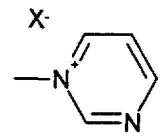 ;

(1l) 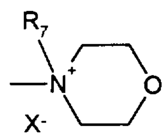 ; (1m) 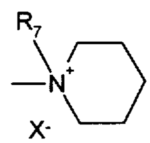 ; or (1n) 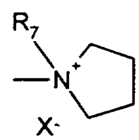 ;

$R_1$, $R_2$, $R_3$, $R_4$, $R_5$ and $R_6$ are each independently of the others hydrogen, $C_1$-$C_{18}$alkyl; $C_5$-$C_7$-cycloalkyl; halogen;
$R_9$ is hydrogen, $C_1$-$C_{12}$alkyl; or $C_5$-$C_7$cycloalkyl;
$R_7$, $R_8$ and $R_{10}$ are each independently of the others hydrogen, $C_1$-$C_{12}$alkyl, $C_5$-$C_7$cycloalkyl, $C_1$-$C_{12}$hydroxyalkyl;
X is halogen; a radical of formula (1s)

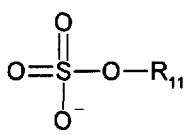 ; (1t) 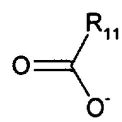 ;

or

(1u) 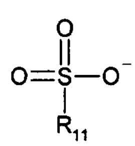 ;

sulfate, phosphate, lactate, citrate, tartrate;
$R_{11}$ is $C_1$-$C_{12}$alkyl; or $C_5$-$C_7$cycloalkyl;
$R_{12}$ and $R_{13}$ are each independently of the other hydrogen; or $C_1$-$C_5$alkyl;
x is from 0 to 10; and
y is from 1 to 20.

The following classes of dispersing agents (component (b)) may be used for the process of the present invention:

($b_1$) alkyl-sugar-derivatives
($b_2$) associative polymers (as described in EP 093 796 A1)
($b_3$) organic acids
($b_4$) emulsifiers
($b_5$) surfactants
($b_6$) phospholipids
($b_7$) polymers, and
($b_8$) other dispersants.

The dispersing agents can be used as single components or as mixtures of one or more of the components ($b_1$) to ($b_8$).

<u>Alkyl-sugar-derivatives (component ($b_1$)):</u>

As alkyl sugar derivatives may be used for instance alkyl polyglucosides or surface-active saccharose esters. The alkyl polyglucoside may consist of a $C_1$-$C_{12}$ester of the compound of formula $C_nH_{2n+1}O(C_6H_{10}O_5)_x$ H, namely an ester formed by reacting a $C_1$-$C_{12}$ acid, such as formic, acetic, propionic, butyric, sulfosuccinic, citric or tartaric acid, with one or more free OH groups on the glucoside moiety ($C_6H_{10}O_5$).

<u>associative polymers (component ($b_2$))</u>

The associative polymers in accordance with the present invention may be anionic, non-ionic, cationic or amphoteric.

Exemplary of the associative anionic polymers are those comprising at least one hydrophilic structural unit, and at least one allyl ether structural unit containing a fatty chain, more particularly those in which the hydrophilic structural unit comprises an ethylenic unsaturated anionic monomer, especially a vinylcarboxylic acid and more preferably an acrylic acid, a methacrylic acid or mixtures thereof, and in which the allyl ether unit containing a fatty chain corresponds to a monomer having the following formula

$$(1) \quad CH_2{=}C(R')CH_2OB_n\text{-}R,$$

in which

- R' is hydrogen; or $CH_3$,
- B represents the ethylenoxy radical,
- n is 0; or is an integer ranging 1 to 100,
- R is a hydrocarbon radical selected from among alkyl, arylalkyl, aryl, alkylaryl or cycloalkyl radicals, having from 8 to 30 carbon atoms, preferably 10 to 24, and more preferably from 12 to 18 carbon atoms.

A more particularly preferred structural unit of formula (I) according to the present invention is a structural unit in which R' is H, n is equal to 10, and R is a stearyl $C_{18}$-radical.

Anionic amphiphilic polymers of this category are described and prepared, via technique for polymerization in emulsion, in EP-0,216,479.

Exemplary of these associative anionic polymers are those polymers prepared from 20% to 60% by weight of acrylic acid and/or of methacrylic acid, from 5% to 60% by weight of lower alkyl(meth)acrylates, from 2% to 50% by weight of allyl ether bearing a fatty chain substituent of formula (I), and from 0% to 1% by weight of a crosslinking agent which is a well known copolymerizable polyethylenic unsaturated monomer such as diallyl phthalate, allyl (meth)acrylate, divinylbenzene, (poly)ethylene glycol dimethacrylate and methylenebisacrylamide. There are most particularly preferred according to the invention.

Among the latter, the crosslinked terpolymers of methacrylic acid, ethyl acrylate, polyethylene glycol (10 EO) stearyl alcohol ether (Steareth 10), in particular those marketed by Allied Colloids, under the trademarks SALCARE SC 80 and SALCARE SC 90 which are aqueous emulsions containing 30% of a crosslinked terpolymer of methacrylic acid, of ethyl acrylate and of steareth-10-allyl ether (40/50/10) are most particularly preferred.

Exemplary associative anionic polymers include those anionic polymers comprising at least one hydrophilic

structural unit of the olefinic unsaturated carboxylic acid type, and at least one hydrophobic structural unit exclusively of the $C_{10}$-$C_{30}$-alkyl ester of unsaturated carboxylic acid type.

Preferably, these polymers are selected from among those whose hydrophilic structural unit of the olefinic unsaturated carboxylic acid type corresponds to the monomer having the following formula

(II): 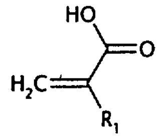

in which

$R_1$ is hydrogen; or $CH_3$ or $C_2H_5$, namely, acrylic acid, methacrylic acid or ethacrylic acid structural units, and whose hydrophobic structural unit of the ($C_{10}$-$C_{30}$)alkyl ester of unsaturated carboxylic acid type corresponds to the monomer having the formula

(III) 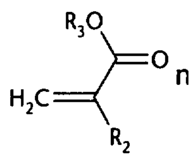

which

$R_2$ is H or $CH_3$ or $C_2H_5$, namely, acrylate, methacrylate or ethacrylate structural units) and preferably H (acrylate units) or $CH_3$ (methacrylate units), and

$R_3$ is a $C_{10}$-$C_{30}$-alkyl, and preferably $C_{12}$-$C_{22}$alkyl.

$C_{10}$-$C_{30}$-alkyl esters of unsaturated carboxylic acids in accordance with the invention include, for example, lauryl acrylate, stearyl acrylate, decyl acrylate, isodecyl acrylate and the corresponding methacrylates, lauryl methacrylate, stearyl methacrylate, decyl methacrylate and isodecyl methacrylate.

Anionic polymers of this type are for example described and prepared according to the methodology set forth in US-3,915,921 and US-4509949.

Among such associative anionic polymers, particularly preferred are those polymers prepared from a mixture of monomers comprising:

(i) essentially acrylic acid,
(ii) an ester having the following formula (III),
in which
$R_2$ is H or $CH_3$ and
$R_3$ is an alkyl radical having from 12 to 22 carbon atoms, and
(iii) a crosslinking agent, which is a well known copolymerizable polyethylenic unsaturated monomer such as diallyl phthalate, allyl (meth)acrylate, divinylbenzene, (poly)ethylene glycol dimethacrylate and methylenebisacrylamide.

Among these associative anionic polymers, more particularly preferred are those comprising 95% to 60% by weight of acrylic acid (hydrophilic structural unit),
4% to 40% by weight of $C_{10}$-$C_{30}$ alkyl acrylate (hydrophobic structural unit), and
0% to 6% by weight of crosslinking polymerizable monomer,
or, alternatively, those comprising
98% to 96% by weight of acrylic acid (hydrophilic structural unit),
1% to 4% by weight of $C_{10}$-$C_{30}$ alkyl acrylate (hydrophobic structural unit) and
0.1% to 0.6% by weight of crosslinking polymerizable monomer such as those described above.

Exemplary of the above polymers are the products marketed by Goodrich under the trademark PEMULEN TR1, PEMULEN TR2, CARBOPOL 1382, and even more preferably PEMULEN TR1, and the product marketed by S. E.P.P.I.C. under the trademark COATEX SX. These are most particularly preferred according to the present invention.

Exemplary associative anionic polymers also include the terpolymers of maleic anhydride/$C_{30}$-$C_{38}$-α-olefin/alkyl maleate such as the product (maleic anhydride/-$C_{30}$-$C_{38}$-α-olefin/isopropyl maleate copolymer) marketed under the trademark PERFORMA V 1608 by Newphase Technologies.

Associative anionic polymers, which are also exemplary, are the acrylic terpolymers comprising:

(a) about 20% to 70% by weight of a carboxylic acid with .alpha., monoethylenic unsaturation,
(b) about 20% to 80% by weight of a nonsurfactant monomer with α-monoethylenic unsaturation different from (a),
(c) about 0.5% to 60% by weight of a nonionic monourethane which is the product of the reaction of a monohydric surfactant with a monoisocyanate with monoethylenic unsaturation, such as those described in EP-A-0,173,109 and more particularly a methacrylic acid/methyl acrylate/dimethyl metaisopropenyl benzyl isocyanate of ethoxylated (40 EO) behenyl alcohol terpolymer in 25% aqueous dispersion.

The nonionic associative polymers according to the invention are preferably selected from among:

(1) celluloses modified by groups comprising at least one fatty chain substituent, including, for example:

(i) the hydroxyethylcelluloses modified by groups comprising at least one fatty chain such as alkyl, arylalkyl or alkylaryl groups, or mixtures thereof, and in which the alkyl groups are preferably $C_8$-$C_{22}$, such as the product NATROSOL PLUS GRADE 330 CS ($C_{16}$-alkyls) marketed by Aqualon, or the product BERMOCOLL EHM 100 marketed by Berol Nobel,
(ii) those modified by polyalkylene glycol ether of alkylphenol groups, such as the product AMERCELL POLYMER HM-1500 (polyethylene glycol (15) ether of nonylphenol) marketed by Amerchol;

(2) the hydroxypropylguars modified by groups comprising at least one fatty chain such as the product ESAFLOR HM 22 ($C_{22}$-alkyl chain) marketed by Lamberti, the products RE 210-18 ($C_{14}$-alkyl chain) and RE 205-1 ($C_{20}$-alkyl chain) marketed by Rhodia;
(3) the polyether/polyurethanes comprising in their chain both hydrophilic sequences which are typically of a polyoxyethylenated nature and hydrophobic sequences which may be aliphatic chains alone and/or cycloaliphatic and/or aromatic chains;
(4) the copolymers of vinylpyrrolidone and of hydrophobic monomers having a fatty chain, for example:

(i) the products ANTARON V216 or GANEX V216 (vinylpyrrolidone/hexadecene copolymer) marketed by I.S.P.,
(ii) the products ANTARON V220 or GANEX V220 (vinylpyrrolidone/eicosene copolymer) marketed by I.S.P.;

(5) the copolymers of $C_1$-$C_6$alkyl methacrylates or acrylates and of amphiphilic monomers comprising at least one fatty chain such as, for example, the oxyethylenated stearyl acrylate/methyl acrylate copolymer marketed by Goldschmidt under the trademark ANTIL 208;
(6) the copolymers of hydrophilic methacrylates or acrylates and of hydrophobic monomers comprising at least one fatty chain such as, for example, the polyethylene glycol methacrylate/lauryl methacrylate copolymer.

Preferably, the polyether/polyurethanes comprise at least two lipophilic hydrocarbon chains, having from $C_6$ to $C_{30}$ carbon atoms, separated by a hydrophilic sequence; the hydrocarbon chains may be pendent chains or chains at the end of a hydrophilic sequence. In particular, one or more pendent chains are envisaged. In addition, the polymer may comprise a hydrocarbon chain at one end or at both ends of a hydrophilic sequence.

The polyether/polyurethanes may be polyblocks, in particular in triblock form. The hydrophobic sequences may be at each end of the chain (for example: triblock copolymer with hydrophilic central sequence) or distributed both at the ends and along the chain (polyblock copolymer for example). These same polymers may also be in the form of graft units or may be star-shaped.

Preferably, the associative nonionic polyether/polyurethanes are triblock copolymers whose hydrophilic sequence is a polyoxyethylenated chain comprising from 50 to 1,000 oxyethylenated groups. Nonionic polyether/polyurethanes comprise a urethane bond between the hydrophilic sequences, hence the origin of the name.

By extension, those whose hydrophilic sequences are linked by other chemical bonds to the lipophilic sequences are also included among the associative nonionic polyether/polyurethanes.

Exemplary associative nonionic polyether/polyurethanes according to the invention include the $C_{16}$-$C_{20}$-EO-polymer marketed by Servo Delden (under the trademark SER-AD FX1100, a molecule with a urethane function and a weight-average molecular weight of 1,300), EO being an oxyethylenated unit. As associative polymer, also exemplary is Rheolate 205 with a urea function marketed by Rheox or, alternatively, Rheolate 208, 204 or 212, as well as Acrysol

RM 184, Aculyn 44 and Aculyn 46 marketed by Rohm & Haas.

Also exemplary is the product ELFACOS T210 containing a $C_{12-14}$ alkyl chain and the product ELFACOS T212 containing a $C_{18}$ alkyl chain, marketed by AKZO.

The product DW 1206B marketed by Rohm & Haas containing a $C_{20}$ alkyl chain and with a urethane bond, containing 20% dry solids content in water, is also exemplary.

Solutions or dispersions of these polymers, in particular, in water or in an aqueous/alcoholic medium, can be used. Exemplary of such polymers are SER-AD FX1010 and SER-AD 1035 marketed by Huls, Rheolate 255, Rheolate 278 and Rheolate 244 marketed by Rheox. The products DW 1206F and DW 1206J are also intended.

The polyurethanes which are suitable according to the invention are, in particular, those described in the article by G. Fonnum, J. Bakke and Fk. Hansen, Colloid Polym. Sci., 271, 380, 389 (1993).

The cationic associative polymers according to the present invention are preferably selected from among the quaternized cellulose derivatives and the polyacrylates with amine-containing side groups.

The quaternized cellulose derivatives include, in particular:

(i) the quaternized celluloses modified by groups comprising at least one fatty chain, such as the alkyl, arylalkyl or alkylaryl groups having at least 8 carbon atoms, or mixtures thereof;
(ii) the quaternized hydroxyethylcelluloses modified by groups comprising at least one fatty chain, such as the alkyl, arylalkyl or alkylaryl groups having at least 8 carbon atoms, or mixtures thereof.

The polyacrylates with amine-containing side groups, quaternized or otherwise, contain, for example, hydrophobic groups of the steareth 20 type (polyoxyethylenated (20) stearyl akohol).

The alkyl radicals borne by the above quaternized celluloses or hydroxyethylcelluloses preferably have from 8 to 30 carbon atoms. The aryl radicals are preferably phenyl, benzyl, naphthyl or anthryl radicals.

Exemplary quaternized alkylhydroxyethylcelluloses containing $C_8$-$C_{30}$ fatty chains are the products QUATRISOFT LM 200, QUATRISOFT LM-X 529-18-A, QUATRISOFT LM-X 529-18B ($C_{12}$-alkyl) and QUATRISOFT LM-X 529-8 ($C_{18}$-alkyl) marketed by Amerchol and the products CRODACEL QM, CRODACEL QL ($C$-$_{12}$alkyl) and CRODACEL QS ($C_{18}$-alkyl) marketed by Croda.

Exemplary polyacrylates with amine-containing side chains are the polymers 8781-121 B or 9492-103 marketed by National Starch.

Exemplary of the associative amphoteric polymers of the invention are the branched or unbranched, crosslinked or non-crosslinked, amphoteric polymers which are prepared by copolymerization:

(1) of at least one monomer of formula

(IVa) 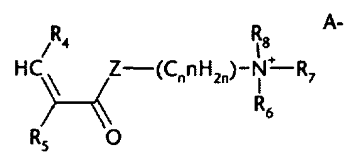

or

(IVb) 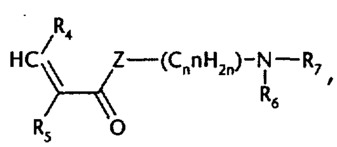

in which
$R_4$ and $R_5$, which may be identical or different, are each a hydrogen atom or a methyl radical,
$R_6$, $R_7$ and $R_8$, which may be identical or different, are each a linear or branched alkyl radical having from 1 to 30 carbon atoms,

- Z  is an NH group or an oxygen atom,
- n  is an integer ranging from 2 to 5,
- $A^-$  is an anion derived from an organic or inorganic acid, such as a methosulfate anion, or a halide such as chloride or bromide;

(2) of at least one monomer of formula

(V) 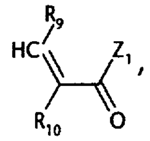

in which

$R_9$ and $R_{10}$, which may be identical or different, and each a hydrogen atom or a methyl radical, and
$Z_1$ is an OH group or a group $NHC(CH_3)_2CH_2SO_3H$;

(3) of at least one monomer of formula

(VI) 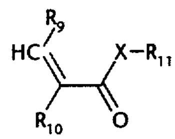

in which

$R_9$ and $R_{10}$, which may be identical or different, are each a hydrogen atom or a methyl radical, X is an oxygen or nitrogen atom and $R_{11}$ is a linear or branched alkyl radical having from 1 to 30 carbon atoms; and

(4) optionally, at least one crosslinking or branching agent; with the proviso that at least one of the monomers of formula (IVa), (IVb) or (VI) includes at least one fatty chain having from 8 to 30 carbon atoms and with the further proviso that said compounds of the monomers of formulae (IVa), (IVb), (V) and (VI) may be quaternized, for example by a $C_1$-$C_4$-alkyl halide or a $C_1$-$C_4$ dialkyl sulfate.

The monomers of formulae (IVa) and (IVb) of the present invention are preferably selected from among:

(i) dimethylaminoethyl methacrylate, dimethylaminoethyl acrylate;
(ii) diethylaminoethyl methacrylate, diethylaminoethyl acrylate;
(iii) dimethylaminopropyl methacrylate, dimethylaminopropyl acrylate;
(iv) dimethylaminopropyl methacrylamide, dimethylaminopropyl acrylamide, optionally quaternized, for example, with a $C_1$-$C_4$-alkyl halide or a $C_1$-$C_4$ dialkyl sulfate.

The monomer of formula (IVa) is preferably acrylamidopropyltrimethylammonium chloride or methacrylamidopropyltrimethylammonium chloride.
The compounds of formula (V) of the present invention are preferably selected from among acrylic acid, methacrylic acid, crotonic acid, 2-methylcrotonic acid, 2-acrylamido-2-methylpropanesulfonic acid and 2-methacrylamido-2-methylpropanesulfonic acid. More preferably, the monomer of formula (V) is acrylic acid.
The monomers of formula (VI) of the present invention are preferably selected from among $C_{12}$-$C_{22}$, and more particularly $C_{16}$-$C_{18}$-alkyl acrylates or methacrylates.
The crosslinking or branching agent is preferably selected from among N,N'-methylenebisacrylamide, triallylmethylammonium chloride, allyl methacrylate, n-methylolacrylamide, polyethylene glycol dimethacrylate, ethylene glycol dimethacrylate, diethylene glycol dimethacrylate, 1,6-hexanediol dimethacrylate and allylsucrose.
The polymers according to the invention may also contain other monomers such as non-ionic monomers and, in particular, $C_1$-$C_4$-alkyl acrylates or methacrylates.
The ratio of the number of cationic charges/anionic charges in these amphoteric polymers is preferably equal to about 1.
The weight-average molecular weight of the associative amphoteric polymers exhibit a weight-average molecular mass greater than 500, preferably of from 10,000 to 10,000,000 and even more preferably from 100,000 to 8,000,000.
Preferably, the associative amphoteric polymers of the invention contain from 1 to 99 mol %, more preferably

from 20 to 95 mol % and even more preferably from 25 to 75 mol % of compound(s) of formula (IVa) or (IVb). They also preferably contain from 1 to 80 mol %, more preferably from 5 to 80 mol % and still more preferably from 25 to 75 mol % of compound(s) of formula (V). The content of compound(s) of formula (VI) preferably ranges from 0.1 to 70 mol %, more preferably from 1 to 50 mol % and even more preferably from 1 to 10 mol %. The crosslinking or branching agent, when it is present, preferably ranges from 0.0001 to 1 mol % and even more preferably from 0.0001 to 0.1 mol %.

Preferably, the molar ratio between the compound(s) of formula (IVa) or (IVb) and the compound(s) of formula (V) ranges from 20:80 to 95:5 and more preferably from 25:75 to 75:25.

Representative associative amphoteric polymers according to the invention are described in WO 98/44,012.

The amphoteric polymers which are particularly preferred according to the invention are acrylic acid/acrylamidopropyltrimethyl-ammonium chloride/stearyl methacrylate copolymers.

According to one particular embodiment of the invention, the associative polymer(s) play the role of emulsifier of the oily phase in the aqueous phase. When the associative polymer is present as sole emulsifier, it is very advantageously present in an amount ranging from 0.1% to 20% of the total weight of the composition, preferably in an amount ranging from 0.5% to 10%.

Organic acids (component $b_3$)

Examples of those fatty acids are capric acid, lauric acid, myristic acid, myristinic acid, palmitinic acid, stearic acid, arachnic acid, behenic acid, caproleinic acid, dodecenic acid, tetradecenic acid, octadecenic acid, oleic acid, eicosenic acid, erucaic acid as well as combinations thereof such as coco fatty acid. The acids can be used in their salt form, e.g. as alkali metal salts such as sodium salts, potassium salts, or metal salts such as Zn and/or aluminium salts or other alkaline reacting , nitrogen-containing organic compounds such as amines or ethoxylated amines.

Emulsifiers (component ($b_4$))

Component ($b_4$) is preferably an emulsifier or emulsifier mixtures forming the preferred O/W structures.

Especially preferred emulsifiers are

- alkali, ammonium and amine salts of fatty acids. Examples of such salts are the lithium, sodium, potassium, ammonium, triethylamine, ethanolamine, diethanolamine or triethanolamine salts. It is preferred to use the sodium, potassium or ammonium ($NR_1R_2R_3$) salts, wherein $R_1$, $R_1$ and $R_1$ are each independently of one another hydrogen, $C_1$-$C_4$alkyl or $C_1$-$C_4$hydroxyalkyl.
- saturated and unsaturated alkyl sulfates, such as sodium docecylsulfate and alkanesulfonates such as sodium dodecanesulfonate;
- salts of colic acid, such as sodium cholate, sodium glycocholate and sodium taurocholate;
- invert soaps (quats), such as zetylpyridinium chloride;
- partial fatty acid esters of sorbitan, such as sorbitan monolaurate;
- sugar esters of fatty acids, such as sucrose monolaurate;
- alkylglucosides, such as n-octylglucoside or n-dodecylglucoside;
- alkylmaltosides, such as n-dodecylmaltoside;
- fatty acid partial glycerides, such as lauric acid monoglyceride;
- $C_8$-$C_{18}$betaines, $C_8$-$C_{24}$alkylamido-$C_1$-$C_4$alkylenebetaines and $C_8$-$C_{18}$sulfobetaines;
- proteins, such as casein;
- polyglycerol esters of fatty acids;
- propylene glycol esters of fatty acids;
- lactates of fatty acids, such as sodium stearoyllactyl-2-lactate;
- fatty alcohol phosphorates.

Emulsifiers of the polyoxyethylene type are very particularly preferred. Examples of such emulsifiers are:

- polyethoxylated sorbitan fatty acid esters, such as polysorbate 80;
- polyethoxylated fatty alcohols, such as oleth-20;
- polyethoxylated fatty acids, such as polyoxyl 20 stearate;
- polyethoxylated vitamin E derivatives, such as vitamin E polyethylene glycol 1000 succinate;
- polyethoxylated lanoline and lanoline derivatives, such as laneth-20;
- polyethoxylated fatty acid partial glycerides, such as diethylene glycol monostearate;
- polyethoxylated alkylphenols, such as ethylphenolpoly(ethylene glycol ether)11;
- sulfuric acid semiester polyethoxylated fatty alcohols and their salts, such as $C_{12}$-$C_{14}$-fatty alcohol ether sulfate-2 EO-sodium salt;

- polyethoxylated fatty amines and fatty acid amides;
- polyethoxylated carbon hydrates
- block polymers of ethylene oxide and propylene oxide, such as poloxamer 188.

Surfactants (component ($b_5$)

Suitable surfactants are anionic, nonionic or zwitterionic and amphoteric synthetic, surface-active substances.
Suitable anionic surface-active substances are:

- sulfates, typically fatty alcohol sulfates, which contain 8 to 18 carbon atoms in the alkyl chain, e.g. sulfated lauryl alcohol;
- fatty alcohol ether sulfates, typically the acid esters or the salts thereof of a polyadduct of 2 to 30 mol of ethylene oxide with 1 mol of a $C_8$-$C_{22}$fatty alcohol;
- the alkali metal salts, ammonium salts or amine salts of $C_8$-$C_{20}$fatty acids, which are termed soaps, typically coconut fatty acid;
- alkylamide sulfates;
- alkylamine sulfates, typically monoethanolamine lauryl sulfate;
- alkylamide ether sulfates;
- alkylaryl polyether sulfates;
- monoglyceride sulfates;
- alkane sulfonates, containing 8 to 20 carbon atoms in the alkyl chain, e.g. dodecyl sulfonate;
- alkylamide sulfonates;
- alkylaryl sulfonates;
- a-olefin sulfonates;
- sulfosuccinic acid derivatives, typically alkyl sulfosuccinates, alkyl ether sulfosuccinates or alkyl sulfosuccinamide derivatives;
- N-[alkylamidoalkyl]amino acids of formula

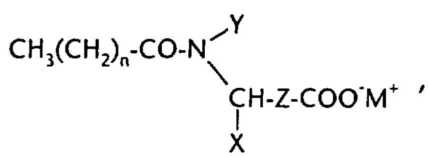

wherein

X is hydrogen, $C_1$-$C_4$alkyl or -$COO^-M^+$; Y is hydrogen or $C_1$-$C_4$alkyl; Z is

$$-(CH_2)_{\overline{m_1-1}} \quad ;$$

$m_1$ is 1 to 5; $n_1$ is an integer from 6 to 18; and M is an alkali metal ion or an amine ion;

- alkyl ether carboxylates and alkylaryl ether carboxylates of formula

(10) $CH_3$-X-Y-A,

wherein

X is a radical :

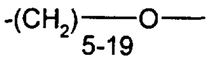 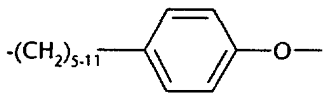 or 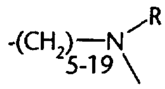

R is hydrogen or $C_1$-$C_4$alkyl, Y is

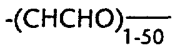 ;

A is:

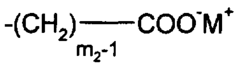

or

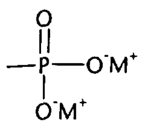 ;

$m_2$ is 1 to 6, and M is an alkali metal cation or an amine cation.

The anionic surfactants used may furthermore be fatty acid methyl taurides, alkylisothionates, fatty acid polypeptide condensates and fatty alcohol phosphoric acid esters. The alkyl radicals in these compounds preferably contain 8 to 24 carbon atoms.

The anionic surfactants are usually obtained in the form of their water-soluble salts, such as the alkali metal, ammonium or amine salts. Typical examples of such salts are lithium, sodium, potassium, ammonium, triethylamine, ethanolamine, diethanolamine or triethanolamine salts. It is preferred to use the sodium or potassium salts or the ammonium-($NR_1R_2R_3$) salts, wherein $R_1$, $R_2$ and $R_3$ are each independently of one another hydrogen, $C_1$-$C_4$alkyl or $C_1$-$C_4$hydroxyalkyl.

Very particularly preferred anionic surfactants in the novel formulation are monoethanolamine lauryl sulfate or the alkali metal salts of fatty alcohol sulfates, preferably the sodium lauryl sulfate, sodium laureth-2 sulfate or sodium cumene sulfonate.

Suitable zwitterionic and amphoteric surfactants are imidazoline carboxylates, alkylamphocarboxy carboxylic acids, alkylamphocarboxylic acids (e.g. lauroamphoglycinate) and N-alkyl-β-aminopropionates or N-alkyl-b-iminodipropionates.

Nonionic surfactants are typically derivatives of the adducts of propylene oxide/ethylene oxide having a molecular weight of 1000 to 15000, fatty alcohol ethoxylates (1-50 EO), alkylphenol polyglycol ethers (1-50 EO), ethoxylated carbohydrates, fatty acid glycol partial esters, typically diethylene glycol monostearate, PEG5 - PEG25 glyceryl stearate, for example PEG-5 glyceryl stearate, PEG15 glyceryl stearate or PEG25 glyceryl stearate; cetearyl octanoate; fatty acid alkanolamides and fatty acid dialkanolamides, fatty acid alkanolamide ethoxylates and fatty acid amine oxides.

Furthermore, the salts of saturated and unsaturated $C_8$-$C_{22}$ fatty acids may be used as solubilizing agents, either by themselves, in admixture with each other or in admixture with the other surface-active substances cited for component (c). Illustrative examples of these fatty acids are typically capric, lauric, myristic, palmitic, stearic, arachic, behenic, dodecenoic, tetradecenoic, octadecenoic, oleic, eicosanic and erucic acid, as well as the technical mixtures of such acids, typically coconut fatty acid. These acids may be obtained in the form of salts, suitable cations being alkali metal cations such as sodium and potassium cations, metal atoms such as zinc atoms and aluminium atoms or nitrogen-containing organic compounds of sufficient alkalinity, typically amines or ethoxylated amines. These salts can also be prepared in situ.

Furthermore, suitable solubilizing agents in the present composition are dihydric alcohols, preferably those

containing 2 to 6 carbon atoms in the alkylene radical, typically ethylene glycol, 1,2- or 1,3-propanediol, 1,3-, 1,4- or 2,3-butanediol, 1,5-pentanediol and 1,6-hexanediol or monohydric alcohol like methanol; ethanol or propanol; and acetone.

Also mixtures of anionic, nonionic, zwitterionic, amphoteric surface-active subatances and one or more of the mono- and/or dihydric alcohols mentioned above can be used for solubilising the antimicrobial agent.

6) Phospholipids (component ($b_6$):

Preferred are phospholipids, hydrated or partially hydrated phospholipids, lysophospholipids, ceramides, or mixtures of these compounds.

A very particularly preferred phospholipid is that of formula

(1) 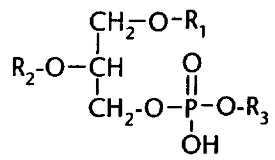

wherein

$R_1$ is $C_{10}$-$C_{20}$acyl;
$R_2$ is hydrogen or $C_{10}$-$C_{20}$acyl
$R_3$ is hydrogen, 2-trimethylamino-1-ethyl, 2-amino-1-ethyl; $C_1$-$C_5$alkyl which is unsubstituted or substituted by one or several carboxy, hydroxy or amino groups; the inositol or glyceryl group; or salts of these compounds.

$C_{10}$-$C_{20}$acyl is preferably straight-chain $C_{10}$-$C_{20}$alkanoyl containing an even number of carbon atoms and straight-chain $C_{10}$-$C_{20}$alkenoyl containing a double bond and an even number of carbon atoms.
Straight-chain $C_{10}$-$C_{20}$alkanoyl containing an even number of carbon atoms is, for example, n-dodecanoyl, n-tetradecanoyl, n-hexadecanoyl or n-octadecanoyl.

Straight-chain $C_{10}$-$C_{20}$alkenoyl containing a double bond and an even number of carbon atoms is, for example, 6-cis- or 6-trans-, 9-cis- or 9-trans-dodecenoyl, -tetradecenoyl, -hexadecenoyl, -octadecenoyl or -eicosenoyl, preferably 9-cis-octa-decenoyl (oleoyl), and also 9,12-cis-octadecadienoyl or 9,12,15-cis-octadecatrienoyl.

A phospholipid of formula (1), wherein $R_3$ is 2-trimethylamino-1-ethyl, is referred to by the trivial name lecithin, and a phospholipid of formula (1), wherein $R_3$ is 2-amino-1-ethyl, by the trivial name cephalin. Suitable are, for example, naturally occurring cephalin or lecithin, e.g. cephalin or lecithin from soybeans or chicken eggs with different or identical acyl groups, or mixtures thereof.

The phospholipid of formula (1) may also be of synthetic origin. The expression "synthetic phospholipid" is used to define phospholipids having uniform composition with respect to $R_1$ and $R_2$. Such synthetic phospholipids are preferably the lecithins and cephalins defined above, wherein the acyl groups $R_1$ and $R_2$ have a defined structure and which are derived from a defined fatty acid having a degree of purity greater than about 95%. $R_1$ and $R_2$ may be identical or different and unsaturated or saturated. Preferably, $R_1$ is saturated, for example n-hexadecanoyl, and $R_2$ is unsaturated, for example 9-cis-octadecenoyl (oleoyl).

The expression "naturally occurring" phospholipid defines a phospholipid that does not have a uniform composition with respect to $R_1$ and $R_2$. Such natural phospholipids are likewise lecithins and cephalins, wherein the acyl groups $R_1$ and $R_2$ are derived from naturally occurring fatty acid mixtures.

The requirement "substantially pure" phospholipid of formula (1) defines a degree of purity of more than 90 % by weight, preferably of more than 95 % by weight of the phospholipid of formula (1), which can be demonstrated by means of suitable determination methods, for example by paper chromatography, thin-layer chromatography, by HPLC or by means of enzymatic colour testing.

In a phospholipid of formula (1), $R_3$ defined as $C_1$-$C_4$alkyl is, for example, methyl or ethyl. Methyl is preferred.

$R_3$ defined as $C_1$-$C_5$alkyl substituted by one or several carboxy, hydroxy or amino groups is, for example, 2-hydroxyethyl, 2,3-dihydroxy-n-propyl, carboxymethyl, 1- or 2-carboxyethyl, dicarboxymethyl, 2-carboxy-2-hydroxyethyl or 3-carboxy-2,3-dihydroxy-n-propyl, 3-amino-3-carboxy-n-propyl or 2-amino-2-carboxy-n-propyl, preferably 2-amino-2-carboxyethyl.

Phospholipids of formula (1) containing these groups can be present in salt form, for example as sodium or potassium salt.

Phospholipids of formula (1), wherein $R_3$ is the inositol or glyceryl group, are known by the names phosphatidylinositol and phosphatidylglycerol.

The acyl radicals in the phospholipids of formula (1) are also customarily known by the names given in brackets:

9-cis-dodecenoyl (lauroleoyl), 9-cis-tetradecenoyl (myristoleoyl), 9-cis-hexadecenoyl (palmitoleoyl), 6-cis-octadecenoyl (petroseloyl), 6-trans-octadecenoyl (petroselaidoyl), 9-cis-octadecenoyl (oleoyl), 9-trans-octadecenoyl (elaidoyl), 9,12-cis-octadecadienoyl (linoleoyl), 9,12,15-cis-octadecatrienoyl (linolenoyl), 11-cis-octadecenoyl (vaccenoyl), 9-cis-eicosenoyl (gadoleoyl), 5,8,11,14-cis-eicosatetraenoyl (arachidonoyl), n-dodecanoyl (lauroyl), n-tetradecanoyl (myristoyl), n-hexadecanoyl (palmitoyl), n-octadecanoyl (stearoyl), n-eicosanoyl (arachidoyl), n-docosanoyl (behenoyl), n-tetracosanoyl (lignoceroyl).

A salt of the phospholipid of formula (1) is preferably cosmetically acceptable. Salts are defined by the existence of salt-forming groups in the substituent $R_3$ and by the free hydroxyl group at the phosphorus atom. The formation of internal salts is also possible. Alkali metal salts, especially the sodium salt, are preferred.

In a particularly preferred embodiment of this invention, purified lecithin from soybeans of the quality LIPOID S 100 or S 75, or a lecithin defined in the monograph USP23/NF 18, is used.

Polymeric dispersant (componet )$b_7$))

Examples for polymers are polyvinylpyrrolidones, which are unmodified or modified through the incorporation of anionic or cationic substituents, in particular those having a molecular weight in the range from 5000 to 60000, more preferably from 10000 to 50000.

Other dispersants (component ($b_8$))

Suitable dispersants for the antimicrobial agents in the present process are:

- acid esters or their salts of alkylene oxide adducts, typically acid esters or their salts of a polyadduct of 4 to 40mol of ethylene oxide with 1 mol of a phenol, or phosphated polyadducts of 6 to 30mol of ethylene oxide with 1 mol of 4-nonylphenol, 1 mol of dinonylphenol or, preferably, with 1mol of compounds which are prepared by addition of 1 to 3mol of unsubstituted or substituted styrenes to 1mol of phenol,
- polystyrene sulfonates,
- fatty acid taurides,
- alkylated diphenyl oxide mono- or disulfonates,
- sulfonates of polycarboxylates,
- the polyadducts of 1 to 60 mol of ethylene oxide and/or propylene oxide with fatty amines, fatty acids or fatty alcohols, each containing 8 to 22 carbon atoms in the alkyl chain, with alkylphenols containing 4 to 16 carbon atoms in the alkyl chain, or with trihydric to hexahydric alkanols containing 3 to 6 carbon atoms, which polyadducts are converted into an acid ester with an organic dicarboxylic acid or with an inorganic polybasic acid,
- ligninsulfonates, and, most preferably,
- formaldehyde condensates such as condensates of ligninsulfonates and/or phenol and formaldehyde, condensates of formaldehyde with aromatic sulfonic acids, typically condensates of ditolyl ether sulfonates and formaldehyde, condensates of naphthalenesulfonic acid and/or naphthol- or naphthylaminesulfonic acids with formaldehyde, condensates of phenolsulfonic acids and/or sulfonated dihydroxydiphenylsulfone and phenols or cresols with formaldehyde and/or urea, as well as condensates of diphenyl oxidedisulfonic acid derivatives with formaldehyde.

For the preparation of the micropigment mixtures it is possible to use any known processes that are suitable for the preparation of microparticles, e.g.:

- wet-grinding with a hard grinding medium, for example zirconium silicate and a protective surfactant or a protective polymer in water or in a suitable organic solvent;
- spray-drying from a suitable solvent, for example aqueous suspensions or suspensions containing organic solvents, or true solutions in water, ethanol, dichloroethane or toluene etc..
- by the expansion according to the RESS process (Rapid Expansion of Supercritical Solutions) of supercritical fluids (e.g. $CO_2$) in which the UV filter(s) is/are dissolved, or the expansion of fluid carbon dioxide together with a solution of one or more UV filters in a suitable organic solvent;
- by reprecipitation from suitable solvents, including supercritical fluids (GASR process = Gas Anti-Solvent Recrystallisation / PCA process = Precipitation with Compressed Antisolvents).

As grinding apparatus for the preparation of the micronised organic UV absorbers according to the invention there may be used, for example, a jet mill, ball mill, vibratory mill or hammer mill, preferably a high-speed mixing mill.
The grinding is preferably carried out using the present combination of components (a) and (b).
The micropigments and mixtures of micropigments so obtained generally have an average particle size of from.0.002 to 10 μm, preferably from 0.0.01 to 3 μm and more especially from 0.02 to 1.0 μm.
By virtue of their lipophilicity, they can satisfactorily be incorporated, alone or together with other soluble organic UV absorbers, into oil-containing and fat-containing cosmetic formulations, such as oils, O/W or W/O emulsions, fatty sticks or gels, in accordance with known methods.
The invention relates also to a UV absorber formulation comprising

(a) 0.1 to 20 % b.w. of a micronised organic UV absorber (a);

(i) 0.1 to 98.8 %, preferably 1 to 40 % b.w., based on the micronised insoluble organic UV absorber, of a dispersing agent (b); and
(ii) 0.1 to 98.8 %, preferably 1 to 40 % b.w., based on the micronised organic UV absorber, of an anionic, cationic or amphoteric UV absorber (c).

The UV absorber formulations according to the present invention can be used for cosmetic preparations The cosmetic preparations are suitable especially as UV filters, that is to say for the protection of organic materials that are sensitive to ultraviolet, especially skin and hair, against the damaging effect of UV radiation.
The cosmetic preparations contain, for example, from 0.1 to 30 % by weight, preferably from 0.1 to 15 % by weight and especially from 0.5 to 10 % by weight, based on the total weight of the composition, of the UV absorber formulation of the present invention and at least one cosmetically tolerable adjuvant.
The cosmetic preparations may be, for example, creams, gels, lotions, alcoholic and aqueous/alcoholic solutions, emulsions, wax/fat compositions, stick preparations, powders or ointments.

Water- and oil-containing emulsions

As water- and oil-containing emulsions (e.g. W/O, O/W, O/W/O and W/O/W emulsions or microemulsions) the preparations contain, for example,
from 0.1 to 30 % by weight, preferably from 0.1 to 15 % by weight and especially from 0.5 to 10 % by weight, based on the total weight of the composition, of the present UV absorber composition,
from 1 to 60 % by weight, especially from 5 to 50 % by weight and preferably from 10 to 35 % by weight, based on the total weight of the composition, of at least one oil component,
from 0 to 30 % by weight, especially from 1 to 30 % by weight und preferably from 4 to 20 % by weight, based on the total weight of the composition, of at least one emulsifier,
from 10 to 90 % by weight, especially from 30 to 90 % by weight, based on the total weight of the composition, of water, and from 0 to 88.9 % by weight, especially from 1 to 50 % by weight, of further cosmetically acceptable adjuvants.

Oil components

The oil phase can be chosen from the following substance groups without limiting the kind of lipophilic ingredient to those substances:

- fatty alcohols like Guerbet alcohols based on fatty alcohols having from 6 to 18, preferably from 8 to 10 carbon atoms including cetyl alcohol, stearyl alcohol, cetearyl alcohol, oleyl alcohol, octyldodecanol, benzoate of C12-C15 alcohols, acetylated lanolin alcohol, etc.
- esters of fatty acids, for example esters of linear $C_6$-$C_{24}$ fatty acids with linear $C_3$-$C_{24}$ alcohols, esters of branched $C_6$-$C_{13}$carboxylic acids with linear $C_6$-$C_{24}$ fatty alcohols, esters of linear $C_6$-$C_{24}$ fatty acids with branched alcohols, especially 2-ethylhexanol, esters of hydroxycarboxylic acids with linear or branched $C_6$-$C_{22}$ fatty alcohols, especially dioctyl malates, esters of linear and/or branched fatty acids with polyhydric alcohols (for example propylene glycol, dimer diol or trimer triol) and/or Guerbet alcohols, for example caproic acid, caprylic acid, 2-ethylhexanoic acid, capric acid, lauric acid, isotridecanoic acid, myristic acid, palmitic acid, palmitoleic acid, stearic acid, isostearic acid, oleic acid, elaidic acid, petroselinic acid, linoleic acid, linolenic acid, elaeostearic acid, arachidic acid, gadoleic acid, behenic acid and erucic acid and technical-grade mixtures thereof (obtained, for example, in the pressure removal of natural fats and oils, in the reduction of aldehydes from Roelen's oxosynthesis or in the dimerisation of unsaturated fatty acids) with alcohols, for example, isopropyl alcohol, caproic alcohol, capryl alcohol, 2-ethylhexyl alcohol, capric alcohol, lauryl alcohol, isotridecyl alcohol, myristyl alcohol, cetyl alcohol, palmoleyl alcohol,

stearyl alcohol, isostearyl alcohol, oleyl alcohol, elaidyl alcohol, petroselinyl alcohol, linoyl alcohol, linolenyl alcohol, elaeostearyl alcohol, arachidyl alcohol, gadoleyl alcohol, behenyl alcohol, erucyl alcohol and brassidyl alcohol and technical-grade mixtures thereof (obtained, for example, in the high-pressure hydrogenation of technical-grade methyl esters based on fats and oils or aldehydes from Roelen's oxosynthesis and as monomer fractions in the dimerisation of unsaturated fatty alcohols).

Examples of such ester oils are isopropylmyristate, isopropylpalmitate, isopropylstearate, isopropyl isostearate, isopropyloleate, n-butylstearate, n-hexyllaurate, n-decyloleate, isooctylstearate, iso-nonylstearate, isononyl isononanoate, 2-ethylhexylpalmitate, 2-hexyllaurate, 2-hexyldecylstearate, 2-octyldodecylpalmitate, oleyloleate, oleylerucate, erucyloleate, erucylerucate, cetearyl octanoate, cetyl palmitate, cetyl stearate, cetyl oleate, cetyl behenate, cetyl acetate, myristyl myristate, myristyl behenate, myristyl oleate, myristyl stearate, myristyl palmitate, myristyl lactate, propylene glycol dicaprylate/caprate, stearyl heptanoate, diisostearyl malate, octyl hydroxystearate, etc.

Further oil components that can be used are

- dicarboxylic acid esters, such as di-n-butyl adipate, di(2-ethylhexyl)-adipate, di(2-ethylhexyl)-succinate and diisotridecyl acelaat, and also diol esters, such as ethylene glycol dioleate, ethylene glycol diisotridecanoate, propylene glycol di(2-ethylhexanoate), propylene glycol diisostearate, propylene glycol dipelargonate, butanediol diisostearate and neopentyl glycol dicaprylate.

- esters of $C_6$-$C_{24}$ fatty alcohols and/or Guerbet alcohols with aromatic carboxylic acids, saturated and/or unsaturated, especially benzoic acid, esters of $C_2$-$C_{12}$dicarboxylic acids with linear or branched alcohols having from 1 to 22 carbon atoms or polyols having from 2 to 10 carbon atoms and from 2 to 6 hydroxy groups

- natural or synthetic triglycerides including glyceryl esters and derivatives

- di- or tri-glycerides, based on C6-C18 fatty acids, modified by reaction with other alcohols (caprylic/capric triglyceride, wheat germ glycerides, etc.)

- fatty acid esters of polyglycerin (polyglyceryl-n such as polyglyceryl-4 caprate, polyglyceryl-2 isostearate, etc.

- castor oil, hydrogenated vegetable oil, sweet almond oil, wheat germ oil, sesame oil, hydrogenated cottonseed oil, coconut oil, avocado oil, corn oil, hydrogenated castor oil, shea butter, cocoa butter, soybean oil, mink oil, sunflower oil, safflower oil, macadamia nut oil, olive oil, hydrogenated tallow, apricot kernel oil, hazelnut oil, borago oil, etc

- waxes including esters of long-chain acids and alcohols as well as compounds having wax-like properties, e.g., carnauba wax, beeswax (white or yellow), lanolin wax, candellila wax, ozokerite, japan wax, paraffin wax, microcrystalline wax, ceresin, cetearyl esters wax, synthetic beeswax,etc.; hydrophilic waxes as cetearyl aAlcohol or partial glycerides.

- pearlescent waxes like alkylene glycol esters, especially ethylene glycol distearate; fatty acid alkanolamides, especially coco fatty acid diethanolamide; partial glycerides, especially stearic acid monoglyceride; esters of polyvalent, unsubstituted or hydroxysubstituted carboxylic acids with fatty alcohols having from 6 to 22 carbon atoms, especially long-chained esters of tartaric acid; fatty substances, for example fatty alcohols, fatty ketones, fatty aldehydes, fatty ethers and fatty carbonates, which in total have at least 24 carbon atoms, especially laurone and distearyl ether; fatty acids, such as stearic acid, hydroxystearic acid or behenic acid, ring-opening products of olefin epoxides having from 12 to 22 carbon atoms with fatty alcohols having from 12 to 22 carbon atoms and/or polyols having from 2 to 15 carbon atoms and from 2 to 10 hydroxy groups, and mixtures thereof.

- hydrocarbon oils

- mineral oil (light or heavy), petrolatum (yellow or white), microcrystalline wax, etc.

- paraffinic and isoparaffinic compounds, hydrogenated isoparaffinic molecules as polydecenes, and polybutene, hydrogenated polyisobutene

- squalane, isohexadecane, isododecane and others from plant and animal kingdom

- silicones or siloxanes (organosubstituted polysiloxanes) like dimethylpolysiloxanes, methylphenylpolysiloxanes, cyclic silicones, amino-, fatty acid-, alcohol-, polyether-, epoxy-, fluorine-, glycoside- and/or alkyl-modified silicone compounds, which at room temperature may be in either liquid or resinous form; linear polysiloxanes ; dimethicone such as Dow Corning® 200 fluid, Mirasil® DM (Rhodia) or dimethiconol; cyclic silicone fluids ; cyclopentasiloxanes volatiles such as Dow Corning® 345 fluid, Silbione® grade, Abil® grade; phenyltrimethicone ; Dow corning® 556 fluid; simethicones, which are mixtures of dimethicones having an average chain length of from 200 to 300 dimethylsiloxane units with hydrogenated silicates. A detailed survey by Todd *et al*. of suitable volatile silicones may in addition be found in Cosm. Toil. 91, 27 (1976).

- fluorinated or perfluorinated oils such as perfluorhexane, dimethylcyclohexane,

- methylcyclopentane (Flutec® grades) or polyperfluoromethylisopropyl ether (Fomblin® grades)

The oil components can be used in an amount of, for example, from 1 to 60 % by weight, especially from 5 to 50 % by weight and preferably from 10 to 35 % by weight, based on the total weight of the composition.

The preparations according to the invention, for example creams, gels, lotions, alcoholic and aqueous/alcoholic solutions, emulsions, wax/fat compositions, stick preparations, powders or ointments, may in addition contain, as further adjuvants and additives, mild surfactants, super-fatting agents, consistency regulators, thickeners, polymers, stabilisers, biogenic active ingredients, deodorising active ingredients, anti-dandruff agents, film formers, swelling agents, further UV light-protective factors, antioxidants, hydrotropic agents, preservatives, insect repellents, self-tanning agents, solubilisers, perfume oils, colourants, bacteria-inhibiting agents and the like.

Super-fatting agents

Substances suitable for use as super-fatting agents are, for example, lanolin and lecithin and also polyethoxylated or acrylated lanolin and lecithin derivatives, polyol fatty acid esters, monoglycerides and fatty acid alkanolamides, the latter simultaneously acting as foam stabilisers.

Surfactants

Examples of suitable mild surfactants, that is to say surfactants especially well tolerated by the skin, include fatty alcohol polyglycol ether sulfates, monoglyceride sulfates, mono- and/or di-alkyl sulfosuccinates, fatty acid isethionates, fatty acid sarcosinates, fatty acid taurides, fatty acid glutamates, $\alpha$-olefin sulfonates, ethercarboxylic acids, alkyl oligoglucosides, fatty acid glucamides, alkylamidobetaines and/or protein fatty acid condensation products, the latter preferably being based on wheat proteins.

Consistency regulators : thickeners and rheology modifiers

As thickeners and rheology modifiers, there come into consideration the groups of silicium dioxide, magnesium silicates, aluminium silicates, polysaccharides or derivatives thereof for example hyaluronic acid, xanthan gum, guar-guar, agar-agar, alginates, Carraghenan, gellan, pectines, or modified cellulose such as hydroxycellulose, hydroxypropylmethylcellulose; in addition polyacrylates or homopolymer of reticulated acrylic acids and polyacrylamides, e.g. the Carbopol range (e.g. Carbopol types 980, 981, 1382, ETD 2001, ETD2020, Ultrez 10; INCI : Carbomer) or Salcare range such as Salcare SC80(Steareth-10 Allyl Ether/Acrylates Copolymer), Salcare SC81 (Acrylates copolymer), Salcare SC91 and Salcare AST(Sodium Acrylates Copolymer/PPG-1 trideceth-6), Sepigel 305(Polyacrylamide/laureth-7), Simulgel NS and Simulgel EG(Hydroxyethyl Acrylate/Sodium Acryloyldimethyl Taurate Copolymer), Stabilen 30 (Acrylates/Vinyl Isodecanoate Crosspolymer), Pemulen TR-1 (Acrylates/C10-30 Alkyl Acrylate Crosspolymer), Luvigel EM (Sodium Acrylates Copolymer), Aculyn 28(Acrylates/Beheneth-25 Methacrylate Copolymer), etc.

Polymers

Suitable cationic polymers are, for example, cationic cellulose derivatives, for example a quaternised hydroxymethyl cellulose obtainable under the name Polymer JR 400® from Amerchol, cationic starches, copolymers of diallylammonium salts and acrylamides, quaternised vinylpyrrolidone/vinyl imidazole polymers, for example Luviquat® (BASF), condensation products of polyglycols and amines, quaternised collagen polypeptides, for example lauryldimonium hydroxypropyl hydrolyzed collagen (Lamequat®L/Grünau), quaternised wheat polypeptides, polyethyleneimine, cationic silicone polymers, for example amidomethicones, copolymers of adipic acid and dimethylaminohydroxypropyldiethylenetriamine (Cartaretin®/Sandoz), copolymers of acrylic acid with dimethyldiallylammonium chloride

(Merquat® 550/Chemviron), polyaminopolyamides, as described, for example, in FR-A-2-252 840, and the crosslinked water-soluble polymers thereof, cationic chitin derivatives, for example of quaternised chitosan, optionally distributed as microcrystals; condensation products of dihaloalkyls, for example dibromobutane, with bisdialkylamines, for example bisdimethylamino-1,3-propane, cationic guar gum, for example Jaguar® C-17, Jaguar@ C-16 from Celanese, quaternised ammonium salt polymers, for example Mirapol® A-15, Mirapol® AD-1, Mirapol® AZ-1 from Miranol.

As anionic, zwitterionic, amphoteric and non-ionic polymers there come into consideration, for example, vinyl acetate/crotonic acid copolymers, vinylpyrrolidone/vinyl acrylate copolymers, vinyl acetate/butyl maleate/isobornyl acrylate copolymers, methyl vinyl ether/maleic anhydride copolymers and esters thereof, uncrosslinked polyacrylic acids and polyacrylic acids crosslinked with polyols, acrylamidopropyltrimethylammonium chloride/acrylate copolymers, octyl acrylamide/methyl methacrylate/tert-butylaminoethyl methacrylate/2-hydroxypropyl methacrylate copolymers, polyvinylpyrrolidone, vinylpyrrolidone/vinyl acetate copolymers, vinylpyrrolidone/dimethylaminoethyl methacrylate/vinyl caprolactam terpolymers and also optionally derivatised cellulose ethers and silicones.

Biogenic active ingredients

Biogenic active ingredients are to be understood as meaning, for example, tocopherol, tocopherol acetate, tocopherol palmitate, ascorbic acid, deoxyribonucleic acid, retinol, bisabolol, allantoin, phytantriol, panthenol, AHA acids, amino acids, ceramides, pseudoceramides, essential oils, plant extracts and vitamin complexes.

Deodorizing active ingredients

As deodorizing active ingredients there come into consideration, for example, antiperspirants, for example aluminium chlorohydrates (see J. Soc. Cosm. Chem. 24, 281 (1973)). Under the trade mark Locron® of Hoechst AG, Frankfurt (FRG), there is available commercially, for example, an aluminium chlorohydrate corresponding to formula $Al_2(OH)_5Cl$ x 2.5 $H_2O$, the use of which is especially preferred (see J. Pharm. Pharmacol. 26, 531 (1975)). Besides the chlorohydrates, it is also possible to use aluminium hydroxyacetates and acidic aluminium/zirconium salts. Esterase inhibitors may be added as further deodorising active ingredients. Such inhibitors are preferably trialkyl citrates, such as trimethyl citrate, tripropyl citrate, triisopropyl citrate, tributyl citrate and especially triethyl citrate (Hydagen® CAT, Henkel KGaA, Düsseldorf/FRG), which inhibit enzyme activity and hence reduce odour formation. Further substances that come into consideration as esterase inhibitors are sterol sulfates or phosphates, for example lanosterol, cholesterol, campesterol, stigmasterol and sitosterol sulfate or phosphate, dicarboxylic acids and esters thereof, for example glutaric acid, glutaric acid monoethyl ester, glutaric acid diethyl ester, adipic acid, adipic acid monoethyl ester, adipic acid diethyl ester, malonic acid and malonic acid diethyl ester and hydroxycarboxylic acids and esters thereof, for example citric acid, malic acid, tartaric acid or tartaric acid diethyl ester. Antibacterial active ingredients that influence the germ flora and kill or inhibit the growth of sweat-decomposing bacteria can likewise be present in the preparations (especially in stick preparations). Examples include chitosan, phenoxyethanol and chlorhexidine gluconate. 5-Chloro-2-(2,4-dichlorophenoxy)-phenol (Irgasan®, Ciba Specialty Chemicals Inc.) has also proved especially effective.

Anti-dandruff agents

As anti-dandruff agents there may be used, for example, climbazole, octopirox and zinc pyrithione. Customary film formers include, for example, chitosan, microcrystalline chitosan, quaternised chitosan, polyvinylpyrrolidone, vinylpyrrolidone/vinyl acetate copolymers, polymers of quaternary cellulose derivatives containing a high proportion of acrylic acid, collagen, hyaluronic acid and salts thereof and similar compounds.

Antioxidants

In addition to the primary light-protective substances it is also possible to use secondary light-protective substances of the antioxidant kind that interrupt the photochemical reaction chain triggered when UV radiation penetrates the skin or hair. Typical examples of such antioxidants are amino acids (e.g. glycine, histidine, tyrosine, tryptophan) and derivatives thereof, imidazoles (e.g. urocanic acid) and derivatives thereof, peptides, such as D,L-carnosine, D-carnosine, L-carnosine and derivatives thereof (e.g. anserine), carotinoids, carotenes (e.g.$\alpha$-carotene, $\alpha$-carotene, lycopene) and derivatives thereof, chlorogenic acid and derivatives thereof, lipoic acid and derivatives thereof (e.g. dihydrolipoic acid), aurothioglycose, propylthiouracil and other thiols (e.g. thioredoxin, glutathione, cysteine, cystine, cystamine and the glycosyl, N-acetyl, methyl, ethyl, propyl, amyl, butyl, lauryl, palmitoyl, oleyl, $\alpha$-linoleyl, cholesteryl and glyceryl esters thereof) and also salts thereof, dilauryl thiodipropionate, distearyl thiodipropionate, thiodipropionic acid and derivatives thereof (esters, ethers, peptides, lipids, nucleotides, nucleosides and salts) and also sulfoximine compounds (e.g. buthionine sulfoximines, homocysteine sulfoximine, buthionine sulfones, penta-, hexa-, hepta-thionine sulfoximine) in very small tolerable amounts, also (metal) chelating agents (e.g. α-hydroxy fatty acids, palmitic acid phytic acid, lactoferrin), α-hydroxy acids (e.g. citric acid, lactic acid, malic acid), humic acid, bile acid, bile extracts, bilirubin, biliverdin, EDTA, EGTA and derivatives thereof, unsaturated fatty acids and derivatives thereof (e.g. α-linolenic acid, linoleic acid, oleic acid), folic acid and derivatives thereof, ubiquinone and ubiquinol and derivatives thereof, vitamin C and derivatives (e.g. ascorbyl palmitate, magnesium ascorbyl phosphate, ascorbyl acetate), tocopherols and derivatives (e.g. vitamin E acetate), vitamin A and derivatives (e.g. vitamin A palmitate) and also coniferyl benzoate of benzoin resin, rutinic acid and derivatives thereof, α-glycosylrutin, ferulic acid, furfurylidene glucitol, carnosine, butyl hydroxytoluene, butyl hydroxyanisole, nordihydroguaiaretic acid, trihydroxybutyrophenone, uric acid and derivatives thereof, mannose and derivatives thereof, superoxide dismutase, N-[3-(3,5-di-tert-butyl-4-hydroxyphenyl)propionyl] sulfanilic acid (and salts thereof, for example the disodium salts), zinc and derivatives thereof (e.g. ZnO, $ZnSO_4$), selenium and derivatives thereof (e.g. selenium methionine), stilbene and derivatives thereof (e.g. stilbene oxide, trans-stilbene oxide) and the derivatives suitable according to the invention (salts, esters, ethers, sugars, nucleotides, nucleosides, peptides and lipids) of those mentioned active ingredients. HALS (="Hindered Amine Light Stabilizers") compounds may also be mentioned. The amount of antioxidants present is usually from 0.001 to 30 % by weight, preferably from 0.01 to 3 % by weight, based on the weight of the UV absorber(s).

Hydrotropic agents

To improve the flow behaviour it is also possible to employ hydrotropic agents, for example ethoxylated or non ethoxylated mono-alcohols, diols or polyols with a low number of C-atoms or their ethers (e.g. ethanol, isopropanol, 1,2-dipropanediol, propyleneglycol, glyerin, ethylene glycol, ethylene glycol monoethylether, ethylene glycol monobutylether, propylene glycol monomethylether, propylene glycol monoethylether, propylene glycol monobutylether, diethylene glycol monomethylether; diethylene glycol monoethylether, diethylene glycol monobutylether and similar products)
The polyols that come into consideration for that purpose have preferably from 2 to 15 carbon atoms and at least two hydroxy groups. The polyols may also contain further functional groups, especially amino groups, and/or may be modified with nitrogen. Typical examples are as follows:

- glycerol;
- alkylene glycols, for example ethylene glycol, diethylene glycol, propylene glycol, butylene glycol, hexylene glycol and also polyethylene glycols having an average molecular weight of from 100 to 1000 Dalton;
- technical oligoglycerol mixtures having an intrinsic degree of condensation of from 1.5 to 10, for example technical diglycerol mixtures having a diglycerol content of from 40 to 50 % by weight;
- methylol compounds, such as, especially, trimethylolethane, trimethylolpropane, trimethylolbutane, pentaerythritol and dipentaerythritol;
- lower alkyl-glucosides, especially those having from 1 to 8 carbon atoms in the alkyl radical, for example methyl and butyl glucoside;
- sugar alcohols having from 5 to 12 carbon atoms, for example sorbitol or mannitol;
- sugars having from 5 to 12 carbon atoms, for example glucose or saccharose;
- amino sugars, for example glucamine;
- dialcohol amines, such as diethanolamine or 2-amino-1,3-propanediol.

Preservatives and Bacteria-inhibiting agents

Suitable preservatives include, for example, methyl-,ethyl-, propyl-, butyl- parabens, benzalkonium chloride, 2-bromo-2-nitro-propane-1,3-diol, dehydroacetic acid, diazolidinyl urea, 2-dichloro-benzyl alcohol, dmdm hydantoin, formaldehyde solution, methyldibromoglutanitrile, phenoxyethanol, sodium hydroxymethylglycinate, imidazolidinyl urea and triclosan, as well as further substance classes listed in the following reference: K.F. Depolo - A Short Textbook of Cosmetology, Chapter 7, Table 7-2, 7-3, 7-4 and 7-5, p 210-219.

Bacteria-inhibiting agents

Typical examples of bacteria-inhibiting agents are preservatives that have a specific action against gram-positive bacteria, such as 2,4,4'-trichloro-2'-hydroxydiphenyl ether (triclosan), chlorhexidine (1,6-di(4-chlorophenyl-biguanido)hexane) or TCC (3,4,4'-trichlorocarbanilide). A large number of aromatic substances and ethereal oils also have antimicrobial properties. Typical examples are the active ingredients eugenol, menthol and thymol in clove oil, mint oil and thyme oil. A natural deodorising agent of interest is the terpene alcohol farnesol (3,7,11-trimethyl-2,6,10-dodecatrien-1-ol), which is present in lime blossom oil. Glycerol monolaurate has also proved to be a bacteriostatic agent.

The amount of the additional bacteria-inhibiting agents present is usually from 0.1 to 2 % by weight, based on the solids content of the preparations.

Perfume oils

There may be mentioned as perfume oils mixtures of natural and/or synthetic aromatic substances. Natural aromatic substances are, for example, extracts from blossom (lilies, lavender, roses, jasmine, neroli, ylang-ylang), from stems and leaves (geranium, patchouli, petitgrain), from fruit (aniseed, coriander, carraway, juniper), from fruit peel (bergamot, lemons, oranges), from roots (mace, angelica, celery, cardamom, costus, iris, calmus), from wood (pinewood, sandalwood, guaiacum wood, cedarwood, rosewood), from herbs and grasses (tarragon, lemon grass, sage, thyme), from needles and twigs (spruce, pine, Scots pine, mountain pine), from resins and balsams (galbanum, elemi, benzoin, myrrh, olibanum, opoponax). Animal raw materials also come into consideration, for example civet and castoreum. Typical synthetic aromatic substances are, for example, products of the ester, ether, aldehyde, ketone, alcohol or hydrocarbon type. Aromatic substance compounds of the ester type are, for example, benzyl acetate, phenoxyethyl isobutyrate, p-tert-butylcyclohexyl acetate, linalyl acetate, dimethylbenzylcarbinyl acetate, phenylethyl acetate, linalyl benzoate, benzyl formate, ethylmethylphenyl glycinate, allylcyclohexyl propionate, styrallyl propionate and benzyl salicylate. The ethers include, for example, benzyl ethyl ether; the aldehydes include, for example, the linear alkanals having from 8 to 18 hydrocarbon atoms, citral, citronellal, citronellyl oxyacetaldehyde, cyclamen aldehyde, hydroxycitronellal, lilial and bourgeonal; the ketones include, for example, isomethylionone and methyl cedryl ketone; the alcohols include, for example, anethol, citronellol, eugenol, isoeugenol, geraniol, linalool, phenyl ethyl alcohol and terpinol; and the hydrocarbons include mainly the terpenes and balsams. It is preferable, however, to use mixtures of various aromatic substances that together produce an attractive scent. Ethereal oils of relatively low volatility, which are chiefly used as aroma components, are also suitable as perfume oils, e.g. sage oil, camomile oil, clove oil, melissa oil, oil of cinnamon leaves, lime blossom oil, juniper berry oil, vetiver oil, olibanum oil, galbanum oil, labolanum oil and lavandin oil. Preference is given to the use of bergamot oil, dihydromyrcenol, lilial, lyral, citronellol, phenyl ethyl alcohol, $\alpha$-hexyl cinnamaldehyde, geraniol, benzyl acetone, cyclamen aldehyde, linalool, boisambrene forte, ambroxan, indole, hedione, sandelice, lemon oil, tangerine oil, orange oil, allyl amyl glycolate, cyclovertal, lavandin oil, muscatel sage oil, $\alpha$-damascone, bourbon geranium oil, cyclohexyl salicylate, vertofix coeur, iso-E-Super, Fixolide NP, evernyl, iraldein gamma, phenylacetic acid, geranyl acetate, benzyl acetate, rose oxide, romillat, irotyl and floramat alone or in admixture with one another.

Colourants

There may be used as colourants the substances that are suitable and permitted for cosmetic purposes, as compiled, for example, in the publication "Kosmetische Färbemittel" of the Farbstoffkommission der Deutschen Forschungsgemeinschaft, Verlag Chemie, Weinheim, 1984, pages 81 to 106. The colourants are usually used in concentrations of from 0.001 to 0.1 % by weight, based on the total mixture.

Other adjuvants

It is furthermore possible for the cosmetic preparations to contain, as adjuvants, anti-foams, such as silicones, structurants, such as maleic acid, solubilisers, such as ethylene glycol, propylene glycol, glycerol or diethylene glycol, opacifiers, such as latex, styrene/PVP or styrene/acrylamide copolymers, complexing agents, such as EDTA, NTA, $\alpha$-alaninediacetic acid or phosphonic acids, propellants, such as propane/butane mixtures, $N_2O$, dimethyl ether, $CO_2$, $N_2$ or air, so-called coupler and developer components as oxidation dye precursors, reducing agents, such as thioglycolic acid and derivatives thereof, thiolactic acid, cysteamine, thiomalic acid or $\alpha$-mercaptoethanesulfonic acid, or oxidising agents, such as hydrogen peroxide, potassium bromate or sodium bromate.

There come into consideration as insect repellents, for example, N,N-diethyl-m-toluamide, 1,2-pentanediol or insect repellent 3535; suitable self-tanning agents are, for example, dihydroxyacetone, erythrulose or mixtures of dihydroxyacetone and erythrulose.

Polymeric beads or hollow spheres as SPF enhancers

The combination of the UV-absorbers, listed in the first chapter, with SPF enhancers, such as non-active ingredients like Styrene/acrylates copolymer, silica beads, spheroidal magnesium silicate, crosslinked Polymethylmethacrylates (PMMA; Micopearl M305 Seppic), can maximize better the UV protection of the sun products.
Holosphere additives (Sunspheres® ISP, Silica Shells Kobo...) deflect radiation and the effective path length of the photon is therefore increased. Ref : Patent EP0893119 A1.

Some beads, as mentioned previously, provide a soft feel during spreading. Moreover, the optical activity of such beads, e.g. micropearl M305, cans modulate skin shine by eliminating reflection phenomena and indirectly may scatter the UV light.

When formulated in O/W emulsions, the preferably amount of such SPF enhancers should represent 1 % to 10% of the total amount of the emulsion.

Cosmetic preparations

Cosmetic formulations according to the invention are contained in a wide variety of cosmetic preparations. There come into consideration, for example, especially the following-preparations:

- skin-care preparations, e.g. skin-washing and cleansing preparations in the form of tablet-form or liquid soaps, soapless detergents or washing pastes,
- bath preparations, e.g. liquid (foam baths, milks, shower preparations) or solid bath preparations, e.g. bath cubes and bath salts;
- skin-care preparations, e.g. skin emulsions, multi-emulsions or skin oils;
- cosmetic personal care preparations, e.g. facial make-up in the form of day creams or powder creams, face powder (loose or pressed), rouge or cream make-up, eye-care preparations, e.g. eyeshadow preparations, mascara, eyeliner, eye creams or eye-fix creams; lip-care preparations, e.g. lipsticks, lip gloss, lip contour pencils, nail-care preparations, such as nail varnish, nail varnish removers, nail hardeners or cuticle removers;
- foot-care preparations, e.g. foot baths, foot powders, foot creams or foot balsams, special deodorants and antiperspirants or callus-removing preparations;
- light-protective preparations, such as sun milks, lotions, creams or oils, sunblocks or tropicals, pre-tanning preparations or after-sun preparations;
- skin-tanning preparations, e.g. self-tanning creams;
- depigmenting preparations, e.g. preparations for bleaching the skin or skin-lightening preparations;
- insect-repellents, e.g. insect-repellent oils, lotions, sprays or sticks;
- deodorants, such as deodorant sprays, pump-action sprays, deodorant gels, sticks or roll-ons;
- antiperspirants, e.g. antiperspirant sticks, creams or roll-ons;
- preparations for cleansing and caring for blemished skin, e.g. synthetic detergents (solid or liquid), peeling or scrub preparations or peeling masks;
- hair-removal preparations in chemical form (depilation), e.g. hair-removing powders, liquid hair-removing preparations, cream- or paste-form hair-removing preparations, hair-removing preparations in gel form or aerosol foams;
- shaving preparations, e.g. shaving soap, foaming shaving creams, non-foaming shaving creams, foams and gels, preshave preparations for dry shaving, aftershaves or aftershave lotions;
- fragrance preparations, e.g. fragrances (eau de Cologne, eau de toilette, eau de parfum, parfum de toilette, perfume), perfume oils or perfume creams;
- cosmetic hair-treatment preparations, e.g. hair-washing preparations in the form of shampoos and conditioners, hair-care preparations, e.g. pretreatment preparations, hair tonics, styling creams, styling gels, pomades, hair rinses, treatment packs, intensive hair treatments, hair-structuring preparations, e.g. hair-waving preparations for permanent waves (hot wave, mild wave, cold wave), hair-straightening preparations, liquid hair-setting preparations, hair foams, hairsprays, bleaching preparations, e.g. hydrogen peroxide solutions, lightening shampoos, bleaching creams, bleaching powders, bleaching pastes or oils, temporary, semi-permanent or permanent hair colourants, preparations containing self-oxidising dyes, or natural hair colourants, such as henna or camomile.

Presentation forms

The final formulations listed may exist in a wide variety of presentation forms, for example:

- in the form of liquid preparations as a W/O, O/W, O/W/O, W/O/W or PIT emulsion and all kinds of microemulsions,
- in the form of a gel,
- in the form of an oil, a cream, milk or lotion,
- in the form of a powder, a lacquer, a tablet or make-up,
- in the form of a stick,
- in the form of a spray (spray with propellent gas or pump-action spray) or an aerosol,
- in the form of a foam, or
- in the form of a paste.

Of special importance as cosmetic preparations for the skin are light-protective preparations, such as sun milks, lotions, creams, oils, sunblocks or tropicals, pretanning preparations or after-sun preparations, also skin-tanning preparations, for example self-tanning creams. Of particular interest are sun protection creams, sun protection lotions, sun protection milk and sun protection preparations in the form of a spray.
Of special importance as cosmetic preparations for the hair are the above-mentioned preparations for hair treatment, especially hair-washing preparations in the form of shampoos, hair conditioners, hair-care preparations, e. g. pretreatment preparations, hair tonics, styling creams, styling gels, pomades, hair rinses, treatment packs, intensive hair treatments, hair-straightening preparations, liquid hair-setting preparations, hair foams and hairsprays. Of special interest are hair-washing preparations in the form of shampoos.
A shampoo has, for example, the following composition: from 0.01 to 5 % by weight of a UV absorber mixture according to the invention, 12.0 % by weight of sodium laureth-2-sulfate, 4.0 % by weight of cocamidopropyl betaine, 3.0 % by weight of sodium chloride, and water ad 100%.
For example, especially the following hair-cosmetic formulations may be used:

$a_1$) spontaneously emulsifying stock formulation, consisting of the UV absorber according to the invention, PEG-6-$C_{10}$oxoalcohol and sorbitan sesquioleate, to which water and any desired quaternary ammonium compound, for example 4 % minkamidopropyl dimethyl-2-hydroxyethylammonium chloride or Quaternium 80 is added;
$a_2$) spontaneously emulsifying stock formulation consisting of the UV absorber according to the invention, tributyl citrate and PEG-20-sorbitan monooleate, to which water and any desired quaternary ammonium compound, for example 4 % minkamidopropyl dimethyl-2-hydroxyethylammonium chloride or Quaternium 80 is added;
b) Quat-doped solutions of the UV absorber according to the invention in butyl triglycol and tributyl citrate;
c) mixtures or solutions of the UV absorber according to the invention with n-alkylpyrrolidone.

The following Examples serve to illustrate the invention but do not limit the invention thereto. The cosmetic active substances are primarily given with their INCI name (INCI = International Norm of Cosmetical Ingredients).

Examples

General

A) Determination of the Particle Size

The particle size is determined with the FO-QELS method (FOQELS = FiberOptische QuasiElastische LichtStreuung). Since only the back scattering is measured at a 180° angle the multiple scattering is insignificant for the particle concentration of one or more percent.
Firstly four samples are taken from the grinding stock and diluted with distilled water on 2% of active ingredient. The diluted suspensions are exposed to ultrasonic treatment for one minute each. Three of the four samples are measured now three times in each case for one minute with FO-QELS, the fourth sample only once, however with a five-minute measuring time.
The particle size distributions are determined in each case with the CONTIN software of the light scattering equipment and evaluated everyone for itself in a EXCEL Spreadsheet as volume-weighted distribution, whereby the form factor is considered.
From the 10 determined d(0,5) - and d(0,9)- values the average values were formed, which represent the relevant measurement. The standard deviation of the d(0,5) - and d(0,9)-values is approx. 10%.

B) Determination of the in vitro SPF

The different dispersions, which differ by quantity and kind of the dispersing agent, were incorporated into standard formulations for the in vitro SPF measurement in such a way that they contained 10% active ingredient containing in each case.
The In vitro SPF values are determined with a Optometrics 290 SPF analyzer according to the method described of Diffey and Robson [2]. Quartz plates glued on Transpore tape (3 M) were used as substrate.

Example 1: Preparation of a Dispersion

250 g of 2,2-methylene bis 6-(2H-benzotriazole-2-yl)-4-(1,1,3,3 tetramethylbutyl)phenol (MBBT) is pre-dispersed in a solution of 75 g of 50% decyl glucoside in 175 g deionized water.
This slurry is then grinded in a dispermat®-pearl mill in presence of 40 ml zirconium oxidgrinding pearls until a particle

size of $d_{0.5}$ = 154 nm is obtained.

Subsequently, 99,4 g of the grounded dispersion are thickened with 0.6 g of a predispersion of 2 parts of xanthan gum in 4 parts propylene glycol.

The obtained thickened dispersion is then incorporated into a screening formulation, which exhibits the following composition:

| Screening formulation | |
|---|---|
| 10.0 % | 2,2'-Methylene-bis-(6-(2H-benzotriazole-2-yl)-4-(1,1,3,3-tetramethylbutyl)-phenol-micropigment |
| 1.5 % | alkylpolyglucoside ($C_{8-16}$alkyl) |
| 1.0 % | NaCl |
| 1.0 % | antifoam agent |
| 0.5 % | xanthan gum |
| 0.4 % | imidazolidinyl urea |
| 0.6 % | 4-hydroxibenzoic acid ester-mixture |
| 85.0 % | deionized water |
| 100.0 % | formulation 1 |

The in-vitro Sun Protection Factor [ SPF ] (2 µl/$cm^2$ of the screening formulation 1 applied on Transpore Tape of the 3M-Company stuck on one on quartz plate) measured with the SPF 290-Analyser of Optometrics amounts to 13.1.

Example 2:

| Composition: | | | |
|---|---|---|---|
| | % | g | Components |
| | 50 | 100 | 2,2'-Methylene-bis-(6-(2H-benzotriazole-2-yl)-4-(1,1,3,3-tetramethylbutyl)-phenol |
| 40 ml | | 145 | grinding medium draison ZYS ∅ 0,3-0,4 mm |
| | 1.875 | 25 | 2-phenyl-benzimidazol-5-sulfonic acid (15% pH 7 with NaOH) |
| | 5.625 WS | 22.5 | decyl glucoside (50%) |
| | 42.5 | 52.5 | Water |
| | 100 | 200 | formulation 2 |

Preparation of the dispersion:

Similar to Example 1 100 g MBBT in a solution of 22.5 g 50% decyl glucoside and 25 g of a solution of 15% 2-phenyl-benzimidazol-5-sulfonic acid (Eusolex® 232 of the company Merck, Darmstadt) adjusted by means of NaOH on pH 7 are pre-dispersed in 52.5 g deionised water.

After the micronisation reaching a $D_{0.5}$ particle size of 158 Nm is obtained. The dispersion as described in example 1 is thickened and a screening formulation is prepared therefrom. The In-vitro SPF (detail see Example. 1) amounts to 14.3.

Example 3:

| Composition: | | | |
|---|---|---|---|
| | % | g | components |
| | 50 | 100 | 2,2'-methylene-bis-(6-(2H-benzotriazole-2-yl)-4-(1,1,3,3-tetramethylbutyl)-phenol |
| 40 ml | | 145 | grinding medium Draison ZYS ∅ 0,3-0,4 mm |

(continued)

| Composition: | | | |
|---|---|---|---|
| | % | g | components |
| | 3.75 | 50 | 2-phenyl-benzimidazol-5-sulfonic acid (15% pH 7 mit NaOH) |
| | 3.75 WS | 15 | decyl glucoside (50%) |
| | 42.5 | 35 | Water |
| | | 200 | formulation 3 |

Preparation of the dispersion :

Similar to Example 2 100 g MBBT in a solution of 15 g 50% decyl glucoside and 50 g of a solution of 15% 2-phenyl-benzimidazol-5-sulfonic acid (Eusolex® 232 of the company Merck, Darmstadt) adjusted by means of NaOH to pH 7 are pre-dispersed in 52.5 g of deionised water.

After micronization a $d_{0.5}$ particle size of 145 nm is obtained. The dispersion is thickened as described in example 1 and a screening formulation is prepared.
The in-vitro SPF (Details see Example 1) is 16.5.

Example 4:

| Composition: | | | |
|---|---|---|---|
| | % | g | Components |
| | 50 | 100 | 2,2'-methylene-bis-(6-(2H-benzotriazole-2-yl)-4-(1,1,3,3-tetramethylbutyl)-phenol |
| 40 ml | | 145 | Grinding medium Draison ZYS ∅ 0,3-0,4 mm |
| | 3.75 WS | 15 | decyl glucoside (50%) |
| | 42.5 | 85 | water |
| | | 200 | formulation 4 |

Preparation of the dispersion:

According to Example 1 100 g MBBT in a solution of 15 g of 50% decyl glucoside are pre-dispersed in 35 g of deionized water (only half of the amount of the dispersing agent of example 1).
After micronization a $D_{0.5}$ particle size of 177 Nm is obtained. The dispersion as described under Example 1 is thickened and a screening formulation is prepared therefrom. The In-vitro SPF (detail see Example 1) amounts to only 7.9.

Overview of particle size $d_{0.5}$ and SPF-data:

Table 1

| Example # | % | dispersant | $d_{0.5}$ [nm] | $d_{0.9}$[nm] | in-vitro SPF |
|---|---|---|---|---|---|
| 1 | 7.500 | decyl glucoside | 154 | 259 | 13.1 |
| 2 | 5.625 1.875 | decyl glucoside 2-phenyl-benzimidazol-5-sulfonic acid | 158 | 240 | 14.3 |
| 3 | 3.750 3.750 | decyl glucoside 2-phenyl-benzimidazol-5-sulfonic acid | 144 | 243 | 16.5 |
| 4 | 3.750 | decyl glucoside | 177 | 327 | 7.9 |

From the results in the table above it is evident that with an optimal particle size (< 160 Nm) and a minimum amount of dispersant (7,5 %) a high SPF can be achieved (Example 1). If the amount of dispersant is strongly reduced, insufficient particle refinement and dispersion stability are obtained, which results in a smaller SPF (Example 4).

However this dispersant reduction can be compensated favorably by appropriate replacement with 2-phenyl-benzimidazol-5-sulfonic acid working as a co-dispersing UV absorber (Examples 2+3).

## Claims

**1.** A method of producing a cosmetic composition comprising a micronised organic UV absorber (a), which method comprises grinding the organic UV absorber, in coarse particle form, in a grinding apparatus, in the presence of a composition, comprising

(i) 0.1 to 99,9%, preferably 1 to 40 % by weight, based on the micronised insoluble organic UV absorber, of a dispersing agent (b); and
(ii) 0.1 to 99.9 % preferably 1 to 40 % by weight, based on the micronised organic UV absorber, of an anionic, cationic or amphoteric UV absorber (c).

**2.** A method according t claim 1, wherein the UV absorber (a) is selected from triazine derivatives, benzotriazole derivatives, amides containing a vinyl group, cinnamic acid derivatives, Fischer base derivatives, diphenyl malonic acid dinitriles, oxalyl amides, camphor derivatives, diphenyl acrylates, para-aminobenzoic acid and salicylates.

**3.** A method according t claim 1 or 2, wherein the triazine derivative corresponds to formula

(1) 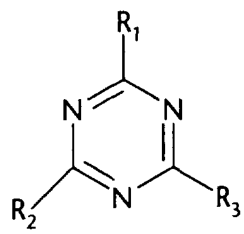 ,

wherein

$R_1$, $R_2$ and $R_3$ are each independently of the others hydrogen; OH; $C_1$-$C_{18}$alkoxy; $-NH_2$; $-NH-R_4$; $-N(R_4)_2$; $-OR_4$,

$R_4$ is $C_1$-$C_5$alkyl; phenyl; phenoxy; anilino; pyrrolo, wherein phenyl, phenoxy, anilino and pyrrolo are unsubstituted or may be substituted by one, two or three OH groups, carboxy, $-CO-NH_2$, $C_1$-$C_5$alkyl or $C_1$-$C_5$alkoxy; a methylidene-camphor group; a group of formula $-(CH=CH)_mC(=O)-OR_4$; a group of formula

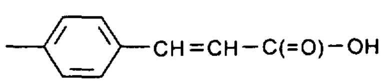

or a corresponding alkali metal, ammonium, mono-, di- or tri-$C_1$-$C_4$alkylammonium, mono-, di- or tri-$C_2$-$C_4$alkanolammonium salt, or a $C_1$-$C_3$alkyl ester thereof; or a radical of formula (1a)

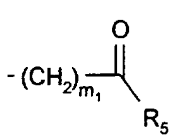 ;

$R_5$ is hydrogen; $C_1$-$C_5$alkyl unsubstituted or substituted by one or more OH groups; $C_1$-$C_5$alkoxy; amino; mono- or di-$C_1$-$C_5$alkylamino; M; a radical of formula

(1b) 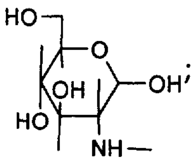 ; (1c) 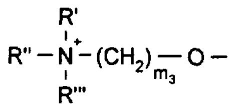 ; (1d) 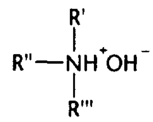 ;

or

(1e) 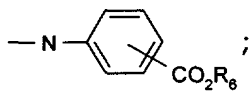 ;

wherein

R', R'' and R''' are each independently of the others $C_1$-$C_{14}$alkyl unsubstituted or substituted by one or more OH groups;

$R_6$ is hydrogen; M; $C_1$-$C_5$alkyl; or a radical of formula $-(CH_2)_{m_2}$-O-$T_1$;

M is a metal cation;

$T_1$ is hydrogen; or $C_1$-$C_8$alkyl;

m is 0 or 1;

$m_2$ is from 1 to 4; and

$m_3$ is from 2 to 14.

**4.** A method according to claim 1 or 2, wherein the organic UV absorber(a) is selected from a triazine derivative of formula

(2) 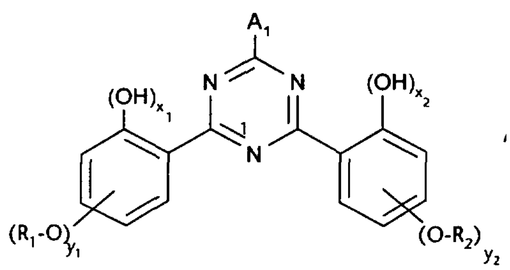 ,

wherein

$R_1$ and $R_8$ are each independently of the other $C_1$-$C_{18}$alkyl; $C_2$-$C_{18}$alkenyl; a radical of formula - $CH_2$-CH(-OH)-$CH_2$-O-$T_1$; or

$R_1$ and $R_2$ are a radical of formula (2a)

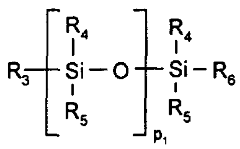 ;

$R_3$ is a direct bond; a straight-chain or branched $C_1$-$C_4$alkylene radical or a radical of formula $-C_{m_1}H_{2m_1}$-O-;

$R_4$, $R_5$ and $R_6$ are each independently of the others $C_1$-$C_{18}$alkyl; $C_1$-$C_{18}$alkoxy or a radical of formula

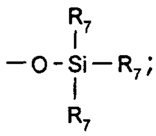

$R_7$ is $C_1$-$C_5$alkyl;
$m_1$ is from 1 to 4;
$p_1$ is from 0 to 5;
$A_1$ is a radical of formula

(2b) 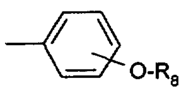 ; (2c) 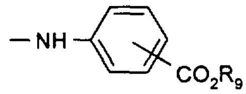 ;

or of formula

(2d) 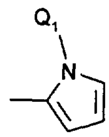 ;

$R_8$ is hydrogen; $C_1$-$C_{10}$alkyl, $-(CH_2CHR_{10}-O)_{n_1}-R_9$; or a radical of formula $-CH_2-CH(-OH)-CH_2-O-T_1$;
$R_9$ is hydrogen; M; $C_1$-$C_5$alkyl; or a radical of formula $-(CH_2)_{m_2}-O-(CH_2)_{m_3}-T_1$;
$R_{10}$ is hydrogen; or methyl;
$T_1$ is hydrogen; or $C_1$-$C_8$alkyl;
$Q_1$ is $C_1$-$C_{18}$alkyl;
M is a metal cation;
$m_2$ and $m_3$ are each independently of the other from 1 to 4;
$n_1$ is from 1 to 16;
$x_1$ and $x_2$, independently from each other are 0 or 1; and
$y_1$ and $y_2$, independently from each other are a number from 1 to 3.

**5.** A method according to claim 1 or 2, wherein the organic UV absorber (a) is a triazine compound of formula

(3) 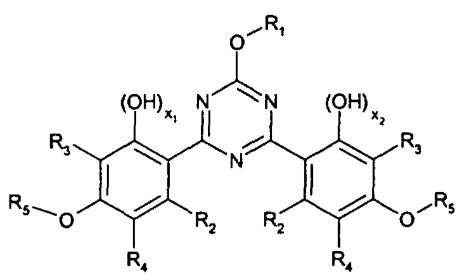

wherein

$R_1$ is $C_1$-$C_{30}$alkyl; $C_2$-$C_{30}$alkenyl; $C_5$-$C_{12}$cycloalkyl unsubstituted or mono- or poly-substituted by $C_1$-$C_5$alkyl; $C_1$-$C_5$alkoxy-$C_1$-$C_{12}$alkyl; amino-$C_1$-$C_{12}$alkyl; $C_1$-$C_5$monoalkylamino-$C_1$-$C_{12}$alkyl; $C_1$-$C_5$dialkylamino-$C_1$-$C_{12}$alkyl; a radical of formula (3a)

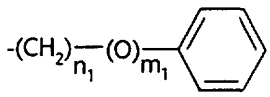

; or (3b)

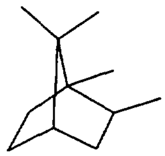

wherein

| | |
|---|---|
| $R_2$, $R_3$ and $R_4$ | are each independently of the others hydrogen, -OH; $C_1$-$C_{30}$alkyl, $C_2$-$C_{30}$alkenyl, |
| $R_5$ | is hydrogen; or $C_1$-$C_5$alkyl; |
| $m_1$ | is 0 or 1; and |
| $n_1$ | is from 1 to 5; and |
| $X_1$ and x2 | are independently from each other 0 or 1. |

**6.** A method according to claim 5, wherein the organic UV absorber (a) is a compound of formula

(3a) 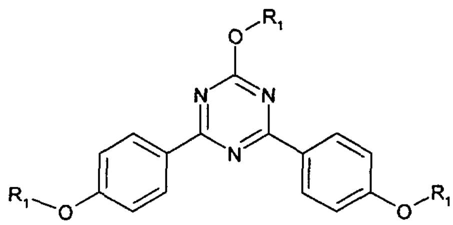 ,

wherein

R is $C_1$-$C_5$alkyl, preferably methyl or ethyl.

**7.** A method according to claim 1 or 2, wherein the organic UV absorber (a) is a benzotriazole compound of formula

(28) 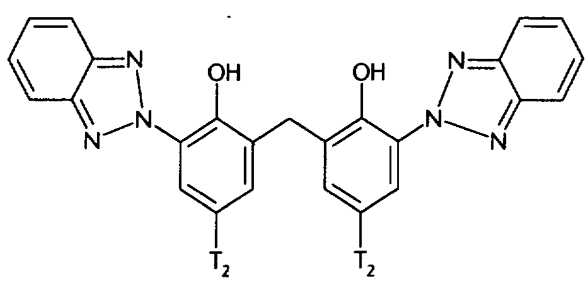 ,

wherein

$T_2$ $C_1$-$C_{10}$alkyl; or phenyl-substituted $C_1$-$C_4$alkyl.

**8.** A method according to any of claims 1 to 7, wherein the dispersing agent (b) is selected from

($b_1$) alkyl-sugar-derivatives

($b_2$) associative polymers
($b_3$) organic acids
($b_4$) emulsifiers
($b_5$) surfactants
($b_6$) phospholipids
($b_7$) polymers, and
($b_8$) other dispersants.

**9.** A method according to any of claims 1 to 8, wherein the dispersing agent (b,) is selected from alkyl polyglucoside having the formula

$$(1) \quad C_nH_{2n+1}(C_6H_{10}O_5)_xH,$$

in which

n is an integer ranging from 8 to 16; and
x is the mean polymerisation level of the glucoside moiety ($C_6H_{10}O_5$) and ranges from 1.4 to 1.6, or an ester thereof.

**10.** A method according to any of claims 1 to 8, wherein the dispersing agent ($b_2$) is selected from an associative polymer comprising the polymerizate of at least one vinylcarboxylic acid and said at least one allyl ether structural unit which comprises a fatty substituent being obtained from a monomer having the formula

$$(I) \qquad CH_2=C(R')CH_2OB_n\text{-}R,$$

in which

R' is H or $CH_3$,
B represents the ethyleneoxy radical,
n is zero or is an integer ranging 1 to 100,
R is a hydrocarbon radical selected from among alkyl, arylalkyl, aryl, alkylaryl or cycloalkyl radicals, having from 8 to 30 carbon atoms

**11.** A method according to according to any of claims 1 to 8, wherein the dispersing agent ($b_2$) is at least one nonionic associative polymer comprising a cellulose selected from the group consisting of a hydroxyethylcellulose modified by at least one fatty alkyl, arylalkyl or alkylaryl radical, or mixture thereof or a hydroxyethylcellulose modified by a polyalkylene glycol ether of an alkylphenol radical.

**12.** A method according to any of claims 1 to 8, wherein the dispersing agent ($b_3$) is selected from alkali, ammonium and amine salts of fatty acids.

**13.** A method according to any of claims 1 to 8, wherein the dispersing agent ($b_4$) is selected from $C_8$-$C_{18}$betaines, $C_8$-$C_{24}$alkylamido-$C_1$-$C_4$alkylenebetaines and $C_8$-$C_{18}$sulfobetaines.

**14.** A method according to any of claims 1 to 8, wherein the dispersing agent ($b_5$) is selected from lauric acid monoglyceride.

**15.** A method according to any of claims 1 to 8, wherein the dispersing agent ($b_6$) is selected from a phospholipid is that of formula

(1) 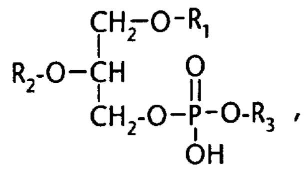 ,

wherein

$R_1$ is $C_{10}$-$C_{20}$acyl;
$R_2$ is hydrogen or $C_{10}$-$C_{20}$acyl
$R_3$ is hydrogen, 2-trimethylamino-1-ethyl, 2-amino-1-ethyl; $C_1$-$C_5$alkyl which is unsubstituted or substituted by one or several carboxy, hydroxy or amino groups; the inositol or glyceryl group; or salts of these compounds.

**16.** A method according to any of claims 1 to 8, wherein the dispersing agent (b,) is selected from a polyvinylpyrrolidone having a molecular weight in the range from 5000 to 60000, more preferably from 10000 to 50000.

**17.** A method according to any one of claims 1 to 16, wherein components ($b_1$)-($b_8$) are used as single components or as mixtures.

**18.** A method according to any one of claims 1 to 17, wherein the anionic UV absorber (c) is selected from toluene, aniline, benzotriazole, benzimidazole, triazine and benzophenone systems.

**19.** A method according to claim 18, wherein the anionic UV absorber (c) is selected from 2-hydroxy-4-methoxy benzophenone-5-sulfonic acid, α-(2-oxoborn-3-ylidene)toluene-4-sulphonic acid and its salts; methyl N,N,N-trimethyl-4-[(4,7,7-trimethyl-3-oxobicyclo[2,2,1]-hept-2-ylidene)methyl]anilinium sulphate; 2- phenyl- 1H- benzimidazole- 5-sulphonic acid, 3, 3'- (1, 4-phenylenedimethylene)bis[7, 7-dimethyl-2-oxo-bicyclo[2.2.1]heptane-1-methanesulfonic acid], disodium phenyl benzimidazole tetrasulfonate, and 1H-benzimidazole-4,6-disulfonic acid, 2,2'-(1,4-phenylene)bis-, disodium salt.

**20.** A method according to claim 18 or 19, wherein the anionic UV absorber (b) is selected from 2-phenyl-benzimidazole-5-sulfonic acid.

**21.** A method according to claim 1, wherein
the organic UV absorber (a) is a benzotriazole compound of formula

(28) 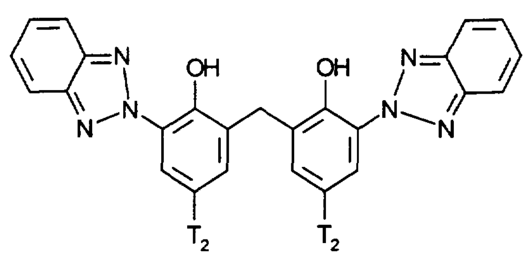 ,

wherein

$T_2$ is as defined for formula (26) and is preferably methyl, tert-butyl or iso-octyl; component (b) is an alkyl polyglucoside having the formula

$$(1) \qquad C_nH_{2n+1}O(C_6H_{10}O_5)_xH,$$

in which
n is an integer ranging from 8 to 16; and
x is the mean polymerization level of the glucoside moiety ($C_6H_{10}O_5$) and ranges from 1.4 to 1.6, or an ester

thereof; and

an anionic UV absorber (c)
is used.

**22.** A method according to claim 21, wherein the anionic UV absorber (c) is selected from 2-hydroxy-4-methoxy benzophenone-5-sulfonic acid, α-(2-oxoborn-3-ylidene)toluene-4-sulphonic acid and its salts; methyl N,N,N-trimethyl-4-[(4,7,7-trimethyl-3-oxobicyclo[2,2,1 ]hept-2-ylidene)methyl]anilinium sulphate; 2-phenyl-1H-benzimidazole-5-sulphonic acid, 3, 3'-(1, 4- phenylenedimethylene)bis[7,7-dimethyl-2-oxo-bicyclo[2.2.1]heptane-1-methanesulfonic acid], disodium phenyl benzimidazole tetrasulfonate, and 1H-benzimidazole-4,6-disulfonic acid, 2,2'-(1,4-phenylene)bis-, disodium salt.

**23.** A method according to claim 21 or 22, wherein
the anionic UV absorber (c) is 2-phenyl-benzimidazole-5-sulfonic acid.

**24.** UV absorber composition, comprising

(a) 0.1 to 20 % b.w. of a micronised organic UV absorber (a);

(i) 0.1 to 98.8 %, preferably 1 to 40 % b.w., based on the micronised insoluble organic UV absorber, of a dispersing agent (b); and
(ii) 0.1 to 98.8 %, preferably 1 to 40 % b.w., based on the micronised organic UV absorber, of an anionic, cationic or amphoteric UV absorber (c).

European Patent Office

# EUROPEAN SEARCH REPORT

Application Number
EP 02 40 5867

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | WO 00 78277 A (CIBA SC HOLDING AG; LUTHER HELMUT (DE)) 28 December 2000 (2000-12-28)<br>* page 1, paragraphs 2-7 *<br>* page 13, paragraph 5 *<br>* page 16, paragraph 3 - page 17, paragraph 1 *<br>* page 19, paragraph 1 *<br>* page 24, paragraphs 2,3 *<br>* page 27, paragraphs 2,3; examples 1,33,36 *<br>--- | 1-10,24 | A61K7/42<br>A61K7/44 |
| X | EP 1 068 866 A (CIBA SC HOLDING AG) 17 January 2001 (2001-01-17)<br>* paragraphs [0005]-[0007],[0029],[0035],[0040]-[0043],[0064]-[0068]; examples 46-48 *<br>--- | 1-10,24 | |
| X | US 6 416 748 B1 (PISSON ANNE-MARIE ET AL) 9 July 2002 (2002-07-09) | 24 | |
| Y | * column 3, line 35 - column 4, line 3 *<br>* column 14, line 43-64 *<br>* column 15, line 1-65; example 1 *<br>--- | 1-10 | TECHNICAL FIELDS SEARCHED (Int.Cl.7)<br>A61K |
| X | US 6 409 998 B1 (PISSON ANNE-MARIE ET AL) 25 June 2002 (2002-06-25) | 24 | |
| Y | * column 1, line 23-31 *<br>* column 3, line 45 - column 4, line 11 *<br>* column 16, line 55-65 *<br>* column 17, line 13 - column 18, line 8; examples 1,2 *<br>--- | 1-10 | |
| | -/-- | | |

~~The present search report has been drawn up for all claims~~

| Place of search | Date of completion of the search | Examiner |
|---|---|---|
| MUNICH | 7 March 2003 | Klaver, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

European Patent Office

# EUROPEAN SEARCH REPORT

Application Number
EP 02 40 5867

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X<br>Y | WO 99 66896 A (CIBA SC HOLDING AG; LUTHER HELMUT (DE); STEHLIN ALBERT (FR)) 29 December 1999 (1999-12-29)<br>* page 2, paragraph 2 - page 3, paragraph 2 *<br>* page 15, paragraph 3 - page 16, paragraph 1 *<br>* page 16, paragraph 7 - page 17, paragraph 1; example 2 *<br>--- | 24<br>1-10 | |
| Y | EP 0 847 750 A (BEIERSDORF AG) 17 June 1998 (1998-06-17)<br>* page 3, line 46-54 *<br>* page 12, line 20-51; examples 4,8,12,16 *<br>--- | 1-10,24 | |
| Y | EP 0 821 939 A (3V SIGMA SPA) 4 February 1998 (1998-02-04)<br>* page 3, line 20 - page 4, line 25; example 3 *<br>--- | 1-10,24 | |
| Y | WO 97 03643 A (CIBA GEIGY AG; LUTHER HELMUT (DE); MINKLEI MARINA (DE); STEHLEIN A) 6 February 1997 (1997-02-06)<br>* page 2, paragraphs 2,3 *<br>* page 12, paragraph 4 *<br>* page 13, paragraphs 6,7 *<br>* page 14, paragraph 3; example 11 *<br>----- | 1-10,24 | TECHNICAL FIELDS SEARCHED (Int.Cl.7) |

~~The present search report has been drawn up for all claims~~

| Place of search | Date of completion of the search | Examiner |
|---|---|---|
| MUNICH | 7 March 2003 | Klaver, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

European Patent Office

Application Number

EP 02 40 5867

## CLAIMS INCURRING FEES

The present European patent application comprised at the time of filing more than ten claims.

- [X] Only part of the claims have been paid within the prescribed time limit. The present European search report has been drawn up for the first ten claims and for those claims for which claims fees have been paid, namely claim(s):

  24

- [ ] No claims fees have been paid within the prescribed time limit. The present European search report has been drawn up for the first ten claims.

## LACK OF UNITY OF INVENTION

The Search Division considers that the present European patent application does not comply with the requirements of unity of invention and relates to several inventions or groups of inventions, namely:

- [ ] All further search fees have been paid within the fixed time limit. The present European search report has been drawn up for all claims.

- [ ] As all searchable claims could be searched without effort justifying an additional fee, the Search Division did not invite payment of any additional fee.

- [ ] Only part of the further search fees have been paid within the fixed time limit. The present European search report has been drawn up for those parts of the European patent application which relate to the inventions in respect of which search fees have been paid, namely claims:

- [ ] None of the further search fees have been paid within the fixed time limit. The present European search report has been drawn up for those parts of the European patent application which relate to the invention first mentioned in the claims, namely claims:

**ANNEX TO THE EUROPEAN SEARCH REPORT ON EUROPEAN PATENT APPLICATION NO.** EP 02 40 5867

This annex lists the patent family members relating to the patent documents cited in the above-mentioned European search report.
The members are as contained in the European Patent Office EDP file on
The European Patent Office is in no way liable for these particulars which are merely given for the purpose of information.

07-03-2003

| Patent document cited in search report | | Publication date | Patent family member(s) | | | Publication date |
|---|---|---|---|---|---|---|
| WO 0078277 | A | 28-12-2000 | AU | 6262600 | A | 09-01-2001 |
| | | | BR | 0011766 | A | 05-03-2002 |
| | | | CN | 1355686 | T | 26-06-2002 |
| | | | WO | 0078277 | A1 | 28-12-2000 |
| | | | EP | 1187598 | A1 | 20-03-2002 |
| | | | JP | 2003502354 | T | 21-01-2003 |
| EP 1068866 | A | 17-01-2001 | AU | 4507000 | A | 18-01-2001 |
| | | | CN | 1281695 | A | 31-01-2001 |
| | | | EP | 1068866 | A2 | 17-01-2001 |
| | | | JP | 2001048764 | A | 20-02-2001 |
| | | | US | 2002155073 | A1 | 24-10-2002 |
| US 6416748 | B1 | 09-07-2002 | FR | 2799965 | A1 | 27-04-2001 |
| | | | AU | 742850 | B2 | 17-01-2002 |
| | | | AU | 6664300 | A | 26-04-2001 |
| | | | BR | 0005141 | A | 29-05-2001 |
| | | | CA | 2324425 | A1 | 22-04-2001 |
| | | | EP | 1093798 | A1 | 25-04-2001 |
| | | | JP | 2001199857 | A | 24-07-2001 |
| US 6409998 | B1 | 25-06-2002 | FR | 2799964 | A1 | 27-04-2001 |
| | | | AT | 232083 | T | 15-02-2003 |
| | | | AU | 744672 | B2 | 28-02-2002 |
| | | | AU | 6664400 | A | 26-04-2001 |
| | | | BR | 0005139 | A | 29-05-2001 |
| | | | CA | 2324056 | A1 | 22-04-2001 |
| | | | EP | 1093796 | A1 | 25-04-2001 |
| | | | JP | 2001199831 | A | 24-07-2001 |
| WO 9966896 | A | 29-12-1999 | AU | 4510999 | A | 10-01-2000 |
| | | | BR | 9911414 | A | 20-03-2001 |
| | | | CN | 1306417 | T | 01-08-2001 |
| | | | WO | 9966896 | A1 | 29-12-1999 |
| | | | EP | 1089706 | A1 | 11-04-2001 |
| | | | JP | 2002518428 | T | 25-06-2002 |
| | | | US | 6521217 | B1 | 18-02-2003 |
| EP 0847750 | A | 17-06-1998 | DE | 19651478 | A1 | 18-06-1998 |
| | | | EP | 0847750 | A2 | 17-06-1998 |
| | | | JP | 10175839 | A | 30-06-1998 |
| EP 0821939 | A | 04-02-1998 | EP | 0821939 | A1 | 04-02-1998 |
| WO 9703643 | A | 06-02-1997 | AT | 196730 | T | 15-10-2000 |
| | | | AU | 699875 | B2 | 17-12-1998 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 02 40 5867

This annex lists the patent family members relating to the patent documents cited in the above-mentioned European search report.
The members are as contained in the European Patent Office EDP file on
The European Patent Office is in no way liable for these particulars which are merely given for the purpose of information.

07-03-2003

| Patent document cited in search report | Publication date | Patent family member(s) | Publication date |
|---|---|---|---|
| WO 9703643 A | | AU 6519996 A | 18-02-1997 |
| | | BR 9609538 A | 23-02-1999 |
| | | CA 2227004 A1 | 06-02-1997 |
| | | DE 69610559 D1 | 09-11-2000 |
| | | DE 69610559 T2 | 31-05-2001 |
| | | DK 840595 T3 | 08-01-2001 |
| | | WO 9703643 A1 | 06-02-1997 |
| | | EP 0840595 A1 | 13-05-1998 |
| | | ES 2151670 T3 | 01-01-2001 |
| | | GB 2303549 A | 26-02-1997 |
| | | IL 122788 A | 19-03-2001 |
| | | JP 2000501064 T | 02-02-2000 |
| | | NZ 313189 A | 29-07-1999 |
| | | PT 840595 T | 30-03-2001 |
| | | US 5980872 A | 09-11-1999 |

EPO FORM P0459

For more details about this annex : see Official Journal of the European Patent Office, No. 12/82